# EUROPEAN PATENT APPLICATION

(11) **EP 4 650 446 A1**
(43) Date of publication of application: **19.11.2025**
(21) Application number: 23922422.3
(22) Date of filing: 23.11.2023
(51) Int. Cl.: C12N 15/113, A61K 31/713, A61P 35/00, A61P 31/20

(54) **SIRNA FOR INHIBITING EXPRESSION OF PROGRAMMED CELL DEATH 1 LIGAND 1 GENE, CONJUGATE THEREOF, PHARMACEUTICAL COMPOSITION THEREOF, AND USE THEREOF**

(30) Priority: 17.02.2023 CN 202310133516; 02.06.2023 CN 202310651785
(71) Applicant: Suzhou Siran Biotechnology Co., Ltd., Suzhou, Jiangsu 215000 (CN)
(72) Inventor: LIU, Nan, Suzhou, Jiangsu 215000 (CN); ZHANG, Hongli, Suzhou, Jiangsu 215000 (CN)
(74) Representative: Bryers Intellectual Property Ltd
(86) International application number: PCT/CN2023/133563
(87) International publication number: WO 2024/169306

(57) **Abstract**

The present invention relates to an siRNA for inhibiting the expression of the programmed cell death ligand-1 gene, a conjugate thereof and a pharmaceutical composition thereof, and a use thereof. The siRNA comprises a sense strand and an antisense strand. Each nucleotide in the siRNA is independently a modified or unmodified nucleotide. The sense strand comprises a nucleotide sequence selected from one of the nucleotide sequences as set forth in SEQ ID NOs: 1 to 12 or a nucleotide sequence with no more than 3 base mutations thereto. The antisense strand comprises a nucleotide sequence selected from one of the nucleotide sequences as set forth in SEQ ID NOs: 13 to 24 or a nucleotide sequence with no more than 5 base mutations thereto.

## Description

### TECHNICAL FIELD OF THE INVENTION

The present invention belongs to the technical field of biomedicine, and specifically relates to an siRNA for inhibiting expression of programmed cell death ligand-1 gene, a conjugate thereof, a pharmaceutical composition thereof, and a use thereof.

### BACKGROUND OF THE INVENTION

Programmed cell death ligand-1 (Programmed cell death 1 ligand 1, PD-L1), also known as cluster of differentiation 274 (CD274) or B7 homolog 1 (B7-H1), is a 40 kDa type I transmembrane protein encoded by the CD274 gene. PD-L1 is expressed on various tissue cells such as T cells, epithelial cells, and endothelial cells, and its expression is upregulated in many tumors.

PD-L1 is the major ligand for PD-1. The interaction of the ligand PD-L1 with the receptor PD-1 can induce the phosphorylation of the intracellular immunoreceptor tyrosine-based switch motif (ITSM) and immunoreceptor tyrosine-based inhibition motif (ITIM), thereby inhibiting T cell activation and cytokine production. During infection or inflammation in normal tissues, this interaction is important for preventing autoimmunity by maintaining immune response homeostasis. In the tumor microenvironment, this interaction can lead to the inactivation of effector T cells, thereby providing immune escape for tumor cells.

Furthermore, it has been reported (Sun Y et al., Am J Physiol Gastrointest Liver Physiol. 2020 Jan 1;318(1):G162-G173.) that viruses regulate the PD-1/PD-L1 pathway to enhance infectivity. During hepatitis B virus (HBV) infection, HBV can increase the expression of PD-L1 through the PTEN/β-catenin/c-Myc signaling pathway, thereby inhibiting T cell responses and ultimately promoting HBV immune escape. Targeting this signaling pathway is a potential strategy for treating chronic hepatitis B.

Currently, most of the marketed or clinical drugs targeting PD-L1 are antibodies or small molecules, such as Envafolimab, Sugemalimab, Durvalumab, APL-502, CA-170, and the like. The indications under investigation for such drugs are mostly in the field of oncology. They are for systemic administration, have a high incidence of adverse reactions in clinical practice, and have poor safety. There are few applications in the field of hepatitis B treatment. Among the currently marketed drugs, only Envafolimab (ASC22) has conducted clinical trials in the United States for the indication of hepatitis B. Preclinical studies have shown that in a woodchuck model infected with hepatitis B virus, the combination of ASC22 and Entecavir can reduce the level of surface antigen (Balsitis S et al., PLoS One. 2018 Feb 14;13(2):e0190058.). Phase IIa clinical data showed a dose-dependent reduction in HBsAg after a single dose of 0.3, 1.0, or 2.5 mg/kg of ASC22.

Currently, in the nucleic acid field, there is only one oligonucleotide (ASO) drug, RO-9191863 from Roche, targeting PD-L1 in the clinical stage for hepatitis B treatment. Generally, ASOs are slightly inferior to siRNA drugs in terms of efficacy and long-lasting effect, and they have the characteristic of systemic distribution. The liver-specific delivery is not as good as that of siRNA drugs conjugated with liver-targeting molecules. siRNA drugs can be conjugated with liver-targeting molecules to specifically deliver the PD-L1-targeting siRNA drug to the liver, which can specifically down-regulate the level of PD-L1 molecules in the liver and reduce the systemic side effects of small molecule, antibody, and ASO drug forms.

To date, although there are some patents for siRNAs targeting PD-L1, no siRNA drug targeting PD-L1 has been marketed, and no siRNA drug is even in the clinical research stage. Therefore, there is still a need to continuously develop siRNA drugs that can inhibit the expression of the PD-L1 gene.

### SUMMARY OF THE INVENTION

An object of the present invention is to provide an siRNA and modified sequences thereof that can specifically inhibit the expression of the PD-L1 gene in cells. A pharmaceutical composition and an siRNA conjugate comprising the siRNA of the present disclosure can effectively deliver the siRNA of the present disclosure to target tissues and/or cells, thereby showing high potential for druggability in treating or preventing diseases associated with PD-L1 expression.

To achieve the above object, the technical solutions adopted by the present invention are:
A first aspect of the present invention provides an siRNA for inhibiting expression of programmed cell death ligand-1 gene, which comprises a sense strand and an antisense strand. The sense strand comprises a nucleotide sequence selected from one of the nucleotide sequences as set forth in SEQ ID NOs: 1 to 12 or a nucleotide sequence with no more than 3 base mutations thereto. The antisense strand comprises a nucleotide sequence selected from one of the nucleotide sequences as set forth in SEQ ID NOs: 13 to 24 or a nucleotide sequence with no more than 5 base mutations thereto.

According to some embodiments, the sense strand comprises a nucleotide sequence with no more than 2 base mutations, or no more than 1 base mutation, to any one of the nucleotide sequences as set forth in SEQ ID NOs: 1 to 12.

According to some embodiments, the base mutation in the sense strand can be at any position in the nucleotide sequence, for example, at any one, two, or three of the first, second, third, fourth, fifth, sixth, seventh, eighth, ninth, tenth, eleventh, twelfth, thirteenth, fourteenth, fifteenth, sixteenth, seventeenth, eighteenth, or nineteenth position.

According to some embodiments, the antisense strand comprises a nucleotide sequence with no more than 5 base mutations, no more than 4 base mutations, no more than 3 base mutations, no more than 2 base mutations, or no more than 1 base mutation to any one of the nucleotide sequences as set forth in SEQ ID NOs: 13 to 24.

According to some embodiments, the base mutation in the antisense strand can be at any position in the nucleotide sequence, for example, at any one, two, three, four, or five of the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, or twenty-first position.

According to some embodiments, in the 5' to 3' direction, the base mutation in the sense strand is at the 3' end of the nucleotide sequence thereof, and the base mutation in the antisense strand is at any one or more positions within the nucleotide sequence, selected from the 5' end, positions 2 to 8, and the last two nucleotides at the 3' end.

According to some embodiments, the base mutation comprises substitution, insertion, or deletion of a base.

In some embodiments, the modified nucleotide is one or a combination of more than one selected from the group consisting of a 2'-methoxy modified nucleotide, a 2'-fluoro modified nucleotide, a 2'-O-CH₂-CH₂-O-CH₃ modified nucleotide, a 2'-O-CH₂-CH=CH₂ modified nucleotide, a 2'-CH₂-CH₂-CH=CH₂ modified nucleotide, a 2'-deoxynucleotide, a 2'-methoxyethyl modified nucleotide, a phosphorothioate-modified nucleotide, a VP-modified nucleotide, LNA, ENA, cET BNA, UNA, GNA, and SAFE-01, wherein in the structure of SAFE-01, R1 is H, OH, or CH₃, and Base is a natural nucleobase, a modified nucleobase, a universal base, or an H atom.

Further, R1 is CH₃.

In some embodiments, the number of modified nucleotides in the sense strand is one, two, three, four, five, six, seven, eight, nine, ten, eleven, twelve, thirteen, fourteen, fifteen, sixteen, seventeen, eighteen, or nineteen.

In some embodiments, the number of modified nucleotides in the antisense strand is one, two, three, four, five, six, seven, eight, nine, ten, eleven, twelve, thirteen, fourteen, fifteen, sixteen, seventeen, eighteen, nineteen, twenty, or twenty-one.

In some embodiments, all of the nucleotides in the sense strand and the antisense strand are modified nucleotides.

In some embodiments, in the 5' to 3' direction, the nucleotides at positions 7, 8, and 9 of the sense strand are 2'-fluoro modified nucleotides, and the nucleotides at positions 2, 14, and 16 of the antisense strand are 2'-fluoro modified nucleotides.

In some further embodiments, the nucleotides at other positions of the sense strand are 2'-methoxy modified nucleotides.

In some further embodiments, in the 5' to 3' direction, the nucleotide at position 6 of the antisense strand is a 2'-fluoro modified nucleotide or SAFE-01, and the nucleotide at position 7 of the antisense strand is a 2'-methoxy modified nucleotide or SAFE-01.

Preferably, SAFE-01 is Ago, Ggo, Cgo, or Ugo.

In some further embodiments, the nucleotides at other positions of the antisense strand are 2'-methoxy modified nucleotides, wherein the other positions refer to positions other than positions 2, 6, 7, 14, and 16.

In some further embodiments, at least one linkage between the following nucleotides in the siRNA is a phosphorothioate linkage:
the linkage between the 1st nucleotide and the 2nd nucleotide from the 5' end of the sense strand;
the linkage between the 2nd nucleotide and the 3rd nucleotide from the 5' end of the sense strand;
the linkage between the 1st nucleotide and the 2nd nucleotide from the 3' end of the sense strand;
the linkage between the 2nd nucleotide and the 3rd nucleotide from the 3' end of the sense strand;
the linkage between the 1st nucleotide and the 2nd nucleotide from the 5' end of the antisense strand;
the linkage between the 2nd nucleotide and the 3rd nucleotide from the 5' end of the antisense strand;
the linkage between the 1st nucleotide and the 2nd nucleotide from the 3' end of the antisense strand; and
the linkage between the 2nd nucleotide and the 3rd nucleotide from the 3' end of the antisense strand.

In some further embodiments, the 3' end of the sense strand is a phosphorothioate group, and the 5' end of the antisense strand is a VP-modified nucleotide.

In some embodiments, the siRNA is selected from any sequence in Table 2 or Table 3, wherein one sense strand and one antisense strand of a duplex number form one siRNA.

A second aspect of the present invention provides an siRNA conjugate, which comprises one or more of the above-mentioned siRNAs, and a conjugating group conjugated to the siRNA.

In some embodiments, the conjugating group is derived from compound SA51, and the structural formula of compound SA51 is:

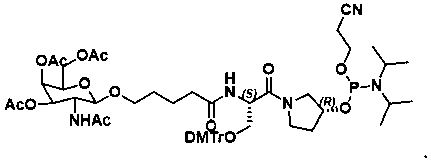

Further, the number of compound SA51 units in the conjugating group is 2 to 4, and the 2 to 4 compound SA51 units are sequentially linked by chemical bonds.

In some embodiments, the conjugating group is linked to the 3' end and/or the 5' end of the sense strand.

In some embodiments, the siRNA conjugate has a structural formula as follows: 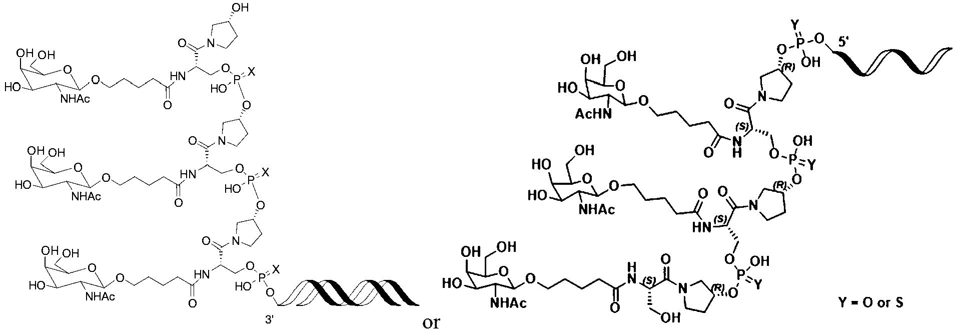 wherein, 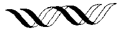 represents a duplex formed by the siRNA of the present disclosure, and X is O or S.

A third aspect of the present invention provides a pharmaceutical composition, which comprises the above-mentioned siRNA or the above-mentioned siRNA conjugate, and a pharmaceutically acceptable carrier or excipient.

In some embodiments, the pharmaceutical composition is for inhibiting programmed cell death ligand-1 gene expression.

A fourth aspect of the present invention provides a use of the above-mentioned siRNA, or the above-mentioned siRNA conjugate, or the above-mentioned pharmaceutical composition for preparing a medicament for treating and/or preventing a disease associated with PD-L1 gene expression.

In some embodiments, the disease is viral hepatitis or cancer.

Further, the viral hepatitis is caused by infection with hepatitis B virus, hepatitis C virus, or hepatitis D virus.

In some embodiments, the above-mentioned siRNA, or the above-mentioned siRNA conjugate, or the above-mentioned pharmaceutical composition is formulated and administered to a subject at a required dose.

In some embodiments, the above-mentioned siRNA, or the above-mentioned siRNA conjugate, or the above-mentioned pharmaceutical composition is administered by subcutaneous injection, intravenous injection, intrathecal injection, or intramuscular injection.

By employing the above technical solutions, the present invention has the following advantages over the prior art:
The human PD-L1 siRNA sequences, conjugates and pharmaceutical compositions thereof disclosed in the present invention have good stability and high PD-L1 mRNA inhibitory activity. The siRNA drug has liver-targeting specificity, better safety compared to small molecule drugs and antibody drugs, and also has a long-lasting effect, allowing for longer dosing intervals in clinical practice and better patient compliance.

### BRIEF DESCRIPTION OF THE DRAWINGS

FIG. 1 is a graph showing the results of an IC50 test of the siRNA of Example 5 in SNU-387 cells;
FIG. 2 is a graph showing the activity results of the siRNA of Example 7 in HDI mice on day 7 and day 28.

### DETAILED DESCRIPTION OF EXEMPLARY EMBODIMENTS

It should be noted that, unless otherwise defined, the technical or scientific terms used in this application shall have the ordinary meaning as understood by a person of ordinary skill in the art.

The experimental methods in the following examples are conventional methods unless otherwise specified. The raw materials, reagents, and the like used in the following examples are commercially available products unless otherwise specified.

### Definitions

In the preceding and subsequent text, a 2'-methoxy modified nucleotide refers to a nucleotide in which the hydroxyl group at the 2' position of the ribose of the nucleotide is replaced by a methoxy group; similarly, a 2'-fluoro modified nucleotide, a 2'-O-CH₂-CH₂-O-CH₃ modified nucleotide, a 2'-O-CH₂-CH=CH₂ modified nucleotide, a 2'-CH₂-CH₂-CH=CH₂ modified nucleotide, a 2'-deoxynucleotide, and a 2'-methoxyethyl modified nucleotide refer to nucleotides in which the hydroxyl group at the 2' position of the ribose of the nucleotide is replaced by the corresponding group, respectively.

A phosphorothioate-modified nucleotide refers to a nucleotide in which one oxygen atom in the phosphodiester bond of the phosphate group of the nucleotide is replaced by a sulfur atom.

A VP-modified nucleotide refers to a nucleotide in which the phosphate group of the nucleotide is replaced by a vinylphosphonate group. In some embodiments, the phosphate group at the 5' end of the antisense strand is replaced by VP.

LNA is as shown in formula (1), ENA is as shown in formula (2), cET BNA is as shown in formula (3), UNA is as shown in in formula (4), GNA is as shown in formula (5), and VP is as shown in formula (6):

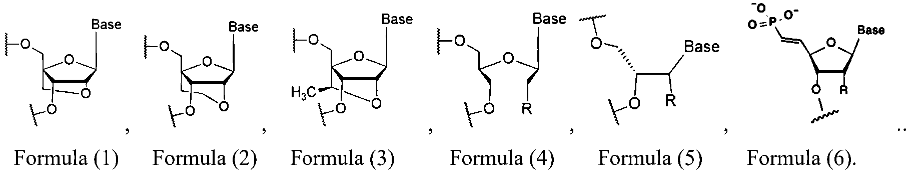

In the above formulas (1) to (6), Base represents a natural nucleobase, a modified nucleobase, or a universal base, such as A, U, G, or C. In the above formulas (4) to (6), R is selected from H, OH, or an alkoxy (O-alkyl) group.

The structural formula of SAFE-01 is 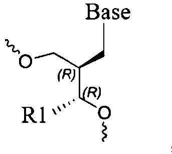 , wherein R1 is H, OH, or CH₃, and Base is a natural nucleobase, a modified nucleobase, a universal base, or an H atom. According to some embodiments, R1 is H. According to some embodiments, R1 is OH. According to some embodiments, R1 is CH₃.

In the preceding and subsequent text, particularly in the description of the methods for preparing the siRNA, pharmaceutical composition, or siRNA conjugate of the present disclosure, unless otherwise specified, the nucleoside monomer refers to a modified or unmodified nucleoside phosphoramidite monomer used in solid-phase phosphoramidite synthesis according to the type and sequence of nucleotides in the siRNA or siRNA conjugate to be prepared. Solid-phase phosphoramidite synthesis is a method well-known to those skilled in the art for RNA synthesis. The nucleoside monomers used in the present disclosure are all commercially available.

In the context of the present disclosure, unless otherwise specified, "conjugation" refers to the covalent connection of two or more chemical moieties, each having a specific function, to each other; accordingly, a "conjugate" refers to a compound formed by the covalent connection of these individual chemical moieties. Further, an "siRNA conjugate" represents a compound formed by the covalent connection of one or more chemical moieties with specific functions to an siRNA. An siRNA conjugate should be understood, depending on the context, as a general term for multiple siRNA conjugates or a specific siRNA conjugate shown by a chemical formula. In the context of the present disclosure, a "conjugating molecule" should be understood as a specific compound that can be conjugated to an siRNA through a reaction, ultimately forming the siRNA conjugate of the present disclosure.

Various hydroxyl protecting groups can be used in the present disclosure. In general, a protecting group renders a chemical functional group insensitive to specific reaction conditions and can be added to and removed from that functional group in a molecule without substantially damaging the rest of the molecule. Representative hydroxyl protecting groups are disclosed in Beaucage et al., Tetrahedron 1992, 48, 2223-2311, and Greene and Wuts, Protective Groups in Organic Synthesis, Chapter 2, 2d ed, John Wiley & Sons, New York, 1991, each of which is incorporated herein by reference in its entirety. In some embodiments, the protecting group is stable under basic conditions but can be removed under acidic conditions. In some embodiments, non-exclusive examples of hydroxyl protecting groups that can be used herein include dimethoxytrityl (DMT), monomethoxytrityl, 9-phenylxanthen-9-yl (Pixyl), and 9-(p-methoxyphenyl)xanthen-9-yl (Mox). In some embodiments, non-exclusive examples of hydroxyl protecting groups that can be used herein include Tr (trityl), MMTr (4-methoxytrityl), DMTr (4,4'-dimethoxytrityl), and TMTr (4,4',4"-trimethoxytrityl).

The pharmaceutically acceptable carrier described in the present disclosure may be a carrier conventionally used in the field of siRNA administration, such as, but not limited to, one or more of magnetic nanoparticles (e.g., Fe₃O₄ or Fe₂O₃ based nanoparticles), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), and poly(2-dimethylaminoethyl methacrylate) (PDMAEMA) and derivatives thereof. The excipient may be one or more of various formulations or compounds conventionally used in the art. For example, the other pharmaceutically acceptable excipients may include at least one of a pH buffer, a protective agent, and an osmotic pressure regulating agent.

As used in this specification, "optional" or "optionally" means that the event or circumstance described thereafter may or may not occur, and the description includes instances where the event or circumstance occurs and instances where it does not.

The term "subject", as used herein, refers to any animal, such as a mammal or a marsupial. Subjects of the present disclosure include, but are not limited to, humans, non-human primates (e.g., rhesus monkeys or other types of macaques), mice, pigs, horses, donkeys, cattle, rabbits, sheep, rats, and any species of poultry.

As used herein, "treating" refers to a method of obtaining a beneficial or desired result, including but not limited to a therapeutic benefit. "Therapeutic benefit" means eradicating or ameliorating the underlying disorder being treated. Furthermore, a therapeutic benefit is obtained by eradicating or ameliorating one or more physiological symptoms associated with the underlying disorder, such that an improvement is observed in the subject, although the subject may still be afflicted with the underlying disorder.

As used herein, "preventing" refers to a method of obtaining a beneficial or desired result, including but not limited to a prophylactic benefit. To obtain a "prophylactic benefit", the siRNA, siRNA conjugate, or pharmaceutical composition may be administered to a subject at risk of developing a particular disease, or to a subject who reports one or more physiological symptoms of the disease, even if a diagnosis of the disease may not have been made.

The technical solutions provided by the present invention will be further described below in conjunction with specific examples. The following examples are only for illustrating the present invention and do not limit the protection scope of the present invention.

### Example 1 Synthesis of siRNA

Herein, if the actual source of a reagent is not given, such a reagent can be obtained from any supplier of molecular biology reagents; and it has a quality/purity standard that meets the requirements for molecular biology applications.

PD-L1 siRNA sequences were synthesized at a 200 nanomole (nmol) scale on a Dr. Oligo 48 synthesizer (Biolytic) using solid support-mediated phosphoramidite chemistry. The solid support was a universal solid support (Biocomma, Shenzhen). The nucleoside monomer raw materials, 2'-F RNA, 2'-O-methyl RNA, and other nucleoside phosphoramidite monomers, were purchased from Hongene Biotech (Shanghai) or GenePharma (Suzhou). The coupling time for all phosphoramidites (50 mM in acetonitrile) was 6 minutes (min), using 5-ethylthio-1H-tetrazole (ETT) as the activator (0.6 M in acetonitrile). A solution of 0.22 M PADS in a 1:1 volume ratio of acetonitrile and trimethylpyridine (Kroma, Suzhou) was used as the sulfurizing reagent, and the sulfurization reaction time was 3 minutes (min). A solution of iodopyridine/water (Kroma) was used as the oxidizing agent, and the oxidation reaction time was 2 minutes (min).

After the solid-phase synthesis was completed, the oligoribonucleotides were cleaved from the solid support by soaking in a 3:1 solution of 28% aqueous ammonia and ethanol at 50°C for 16 hours. Then, after high-speed centrifugation, the supernatant was transferred to another centrifuge tube and evaporated to dryness. The product was purified by C18 reverse-phase chromatography with 0.1 M TEAA and acetonitrile as the mobile phase, and the DMTr was removed using a 3% trifluoroacetic acid solution. The target oligonucleotide was collected, lyophilized, identified as the target product by LC-MS, and then quantified by UV (260 nm).

The resulting single-stranded oligonucleotides were annealed according to an equimolar ratio of two complementary sequences. The final double-stranded siRNA was dissolved in 1X PBS and adjusted to the required concentration for experiments. These monomers are interconnected by 5'-3'-phosphodiester bonds to form oligonucleotides.

### Example 2 Preparation of siRNA conjugate

### I. Preparation of GalNAc targeting moiety

In this example, the synthesis route of compound SA51 (I-1-7) is as follows:

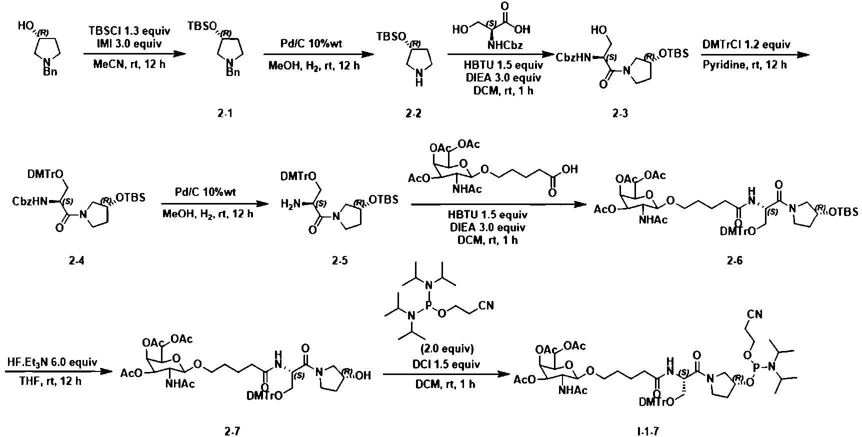

### 1. Preparation of intermediate 2-1

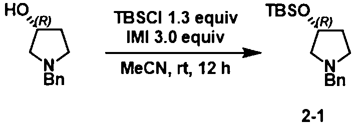

(R)-(+)-N-Benzyl-3-hydroxypyrrolidine (commercially available, purchased from Shanghai Titan Scientific Co., Ltd.) (16.9 mmol, 3.0 g) and imidazole (3.0 equiv, 50.7 mmol, 3.45 g) were placed in a clean and dry reaction flask. 50 mL of acetonitrile was added, and tert-butyldimethylsilyl chloride (1.3 equiv, 21.9 mmol, 3.31 g) was added slowly at room temperature, followed by stirring at room temperature for 12 hours. After the reaction, 100 mL of ethyl acetate was added to the reaction solution, and it was washed with 100 mL of saturated sodium bicarbonate solution and 100 mL of saturated brine. The organic phase was dried, filtered, and concentrated. The resulting crude product was purified by silica gel column chromatography (petroleum ether/ethyl acetate = 20/1 - 5/1) to obtain compound 2-1 as a colorless oil (4.9 g, 16.8 mmol, 99% yield). Molecular formula of compound 2-1: C₁₇H₂₉ONSi, molecular weight: 291.2, LC-MS found: 292.4 (M+H).

### 2.2 Preparation of intermediate 2-2

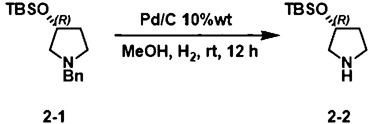

Compound 2-1 (16.8 mmol, 4.9 g) was placed in a clean and dry reaction flask. 100 mL of methanol was added, and palladium on carbon (wet basis, 10% Pd/C) (10% wt, 490.0 mg) was added under hydrogen at room temperature, followed by stirring at room temperature for 12 hours. After the reaction, the palladium on carbon was removed by filtration, and the filtrate was concentrated to obtain the crude product, compound 2-2, as a white solid (3.31 g, 16.5 mmol, 98% yield), which was used directly in the next step without purification. Molecular formula of compound 2-2: C₁₀H₂₃ONSi, molecular weight: 201.1, LC-MS found: 202.3 (M+H).

### 2.3 Preparation of intermediate 2-3

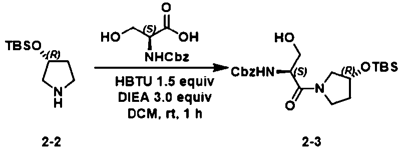

N-Benzyloxycarbonyl-L-serine (commercially available, purchased from Shanghai Titan Scientific Co., Ltd.) (15.0 mmol, 3.58 g) was placed in a clean and dry reaction flask. 100 mL of dichloromethane was added. At room temperature, benzotriazole-N,N,N',N'-tetramethyluronium hexafluorophosphate (1.5 equiv, 22.5 mmol, 8.53 g), compound 2-2 (1.1 equiv, 16.5 mmol, 3.31 g), and N,N-diisopropylethylamine (3.0 equiv, 45.0 mmol, 5.78 g) were added, followed by stirring at room temperature for 1 hour. After the reaction, 150 mL of dichloromethane was added to the reaction solution, and it was washed with 150 mL of saturated sodium bicarbonate solution and 150 mL of saturated brine. The organic phase was dried, filtered, and concentrated. The resulting crude product was purified by silica gel column chromatography (petroleum ether/ethyl acetate = 10/1 - 1/3) to obtain compound 2-3 as a white solid (4.5 g, 10.65 mmol, 63% yield over two steps). Molecular formula of compound 2-3: C₂₁H₃₄O₅N₂Si, molecular weight: 422.2, LC-MS found: 423.3 (M+H). ¹H NMR (400 MHz, CDCl₃): δ 7.35-7.29 (m, 5H), 5.96 (dd, J = 14.3, 8.3 Hz, 1H), 5.10 (s, 2H), 4.61 - 4.40 (m, 2H), 3.85 - 3.68 (m, 2H), 3.65 - 3.49 (m, 2H), 3.41 (d, J = 12.7 Hz, 1H), 3.31 (s, 1H), 1.95 (qdd, J = 15.0, 11.8, 5.3 Hz, 2H), 1.77 (s, 1H), 0.86 (s, 9H), 0.06 (d, J = 3.1 Hz, 6H).

### 2.4 Preparation of intermediate 2-4

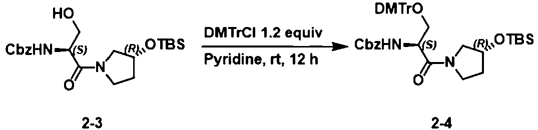

Compound 2-3 (10.65 mmol, 4.5 g) was placed in a clean and dry reaction flask. 100 mL of pyridine was added, and 4,4'-dimethoxytrityl chloride (1.2 equiv, 12.78 mmol, 4.32 g) was added at room temperature, followed by stirring at room temperature for 12 hours. After the reaction, 150 mL of ethyl acetate was added to the reaction solution, and it was washed with 150 mL of saturated sodium bicarbonate solution and 150 mL of saturated brine. The organic phase was dried, filtered, and concentrated. The resulting crude product was purified by silica gel column chromatography (petroleum ether/ethyl acetate = 20/1 - 1/1) to obtain compound 2-4 as a pale yellow oil (7.56 g, 10.43 mmol, 98% yield). Molecular formula of compound 2-4: C₄₂H₅₂O₇N₂Si, molecular weight: 724.3, LC-MS found: 747.4 (M+Na). ¹H NMR (400 MHz, CDCl₃): δ 7.35-7.31 (m, 1H), 7.28 (d, J = 4.7 Hz, 4H), 7.27 - 7.23 (m, 2H), 7.23 - 7.14 (m, 5H), 7.13 - 7.11 (m, 2H), 6.80-6.78 (m, 1H), 6.76 (dd, J = 7.7, 5.4 Hz, 4H), 5.72 (dd, J = 22.7, 8.3 Hz, 1H), 5.08 - 4.99 (m, 2H), 4.69 - 4.59 (m, 1H), 4.35 - 4.30 (m, 1H), 3.73 (dd, J = 4.5, 3.7 Hz, 6H), 3.65 - 3.44 (m, 2H), 3.36 - 3.20 (m, 3H), 1.86 - 1.81 (m, 1H), 1.70 (s, 1H), 0.80 (d, J = 13.1 Hz, 9H), -0.02 (dd, J = 14.9, 4.2 Hz, 6H).

### 2.5 Preparation of intermediate 2-5

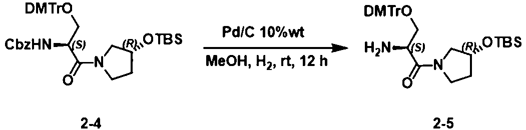

Compound 2-4 (10.43 mmol, 7.56 g) was placed in a clean and dry reaction flask. 100 mL of methanol was added, and palladium on carbon (wet basis, 10% Pd/C) (10% wt, 750.0 mg) was added under hydrogen at room temperature, followed by stirring at room temperature for 12 hours. After the reaction, the palladium on carbon was removed by filtration, and the filtrate was concentrated to obtain the crude product, compound 2-5, as a white solid (6.0 g, 10.22 mmol, 98% yield), which was used directly in the next step without purification. Molecular formula of compound 2-5: C₃₄H₄₆O₅N₂Si, molecular weight: 590.3, LC-MS found: 591.6 (M+H).

### 2.6 Preparation of intermediate 2-6

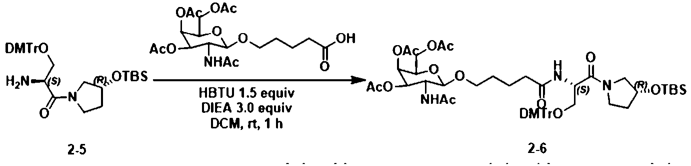

5-(((2R,3R,4R,5R,6R)-3-acetamido-4,5-diacetoxy-6-(acetoxymethyl)tetrahydro-2H-pyran-2-yl)oxy)pentanoic acid (commercially available, purchased from All-Can Pharmaceutical & Chemical Co., Ltd., Suzhou) (2.06 g, 4.62 mmol) was placed in a clean and dry reaction flask. 100 mL of dichloromethane was added. At room temperature, benzotriazole-N,N,N',N'-tetramethyluronium hexafluorophosphate (1.5 equiv, 6.93 mmol, 2.63 g) was added and stirred for ten minutes. Then, compound 2-5 (1.1 equiv, 5.08 mmol, 3.0 g) and N,N-diisopropylethylamine (3.0 equiv, 13.86 mmol, 17.91 g) were added to the reaction system, followed by stirring at room temperature for 1 hour. After the reaction, 150 mL of dichloromethane was added to the reaction solution, and it was washed with 150 mL of saturated sodium bicarbonate solution and 150 mL of saturated brine. The organic phase was dried, filtered, and concentrated. The resulting crude product was purified by silica gel column chromatography (dichloromethane/methanol = 50/1 - 10/1) to obtain compound 2-6 as a white solid (3.58 g, 3.51 mmol, 76% yield). Molecular formula of compound 2-6: C₅₃H₇₃O₁₅N₃Si, molecular weight: 1019.3, LC-MS found: 1018.3 (M-H).

### 2.7 Preparation of intermediate 2-7

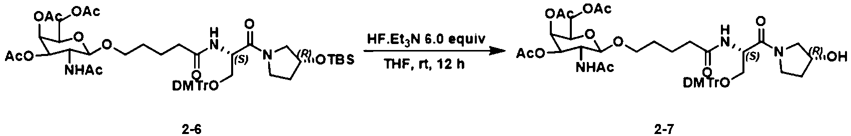

Compound 2-6 (3.51 mmol, 3.58 g) was placed in a clean and dry reaction flask. 50 mL of pyridine was added, and triethylamine hydrofluoride (5.0 equiv, 17.55 mmol, 2.97 g) was added at room temperature, followed by stirring at room temperature for 12 hours. After the reaction, 100 mL of ethyl acetate was added to the reaction solution, and it was washed with 100 mL of saturated sodium bicarbonate solution and 100 mL of saturated brine. The organic phase was dried, filtered, and concentrated. The resulting crude product was purified by silica gel column chromatography (dichloromethane/methanol = 50/1 - 10/1) to obtain compound 2-7 as a pale yellow oil (2.4 g, 2.65 mmol, 76% yield). Molecular formula of compound 2-7: C₄₇H₅₉O₁₅N₃, molecular weight: 905.3, LC-MS found: 904.4 (M-H). ¹H NMR (400 MHz, CDCl₃): δ 7.30 (dd, J = 4.2, 1.9 Hz, 1H), 7.29-7.25 (m, 5H), 7.19-7.15 (m, 4H), 6.85-6.81 (m, 4H), 5.40-5.34 (m, 1H), 5.30 (s, 1H), 5.26 -5.19 (m, 1H), 5.13 (ddd, J = 14.5, 10.9, 3.3 Hz, 1H), 4.85 - 4.78 (m, 1H), 4.75 - 4.71 (m, 1H), 4.68 - 4.61 (m, 1H), 4.50 (d, J = 11.5 Hz, 1H), 4.18 - 4.11 (m, 2H), 4.06 - 3.97 (m, 1H), 3.90 (dt, J = 10.6, 6.8 Hz, 2H), 3.80 (s, 6H), 3.67 - 3.46 (m, 4H), 3.25 - 3.19 (m, 2H), 2.34 - 2.23 (m, 1H), 2.17 - 2.15 (m, 3H), 2.10 - 2.03 (m, 5H), 1.98 (dt, J = 12.9, 2.4 Hz, 4H), 1.76 - 1.61 (m, 4H), 1.37 (t, J = 7.3 Hz, 3H).

### 2.8 Preparation of compound I-1-7

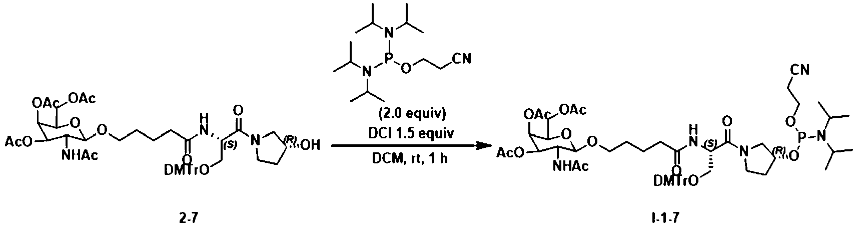

Compound 2-7 (2.65 mmol, 2.4 g) was placed in a clean and dry reaction flask. 50 mL of anhydrous dichloromethane (commercially available, purchased from Shanghai Titan Scientific Co., Ltd.) was added. At room temperature under argon protection, 2-cyanoethyl N,N,N',N'-tetraisopropylphosphorodiamidite (2.0 equiv, 5.3 mmol, 1.6 g) and 4,5-dicyanoimidazole (1.5 equiv, 3.97 mmol, 470.0 mg) were added, and stirring was continued at room temperature for one hour. After the reaction, 50 mL of dichloromethane was added to the reaction solution, and it was washed with 100 mL of saturated sodium bicarbonate solution. The organic phase was dried, filtered, and concentrated. The resulting crude product was purified by C18 reverse-phase column (specification: 30µm; 100 Å, commercially available, purchased from Shanghai Boinn Technology Co., Ltd.) (MeCN : H₂O = 75%:25%) to obtain I-1-7 as a white solid (2.03 g, 1.84 mmol, 70% yield). Molecular formula of compound 1-1-7: C₅₆H₇₆O₁₅N₆P, molecular weight: 1105.5, LC-MS found: 1128.4 (M+Na). ¹H NMR (400 MHz, DMSO-d₆): δ 8.12-8.06 (m, 1H), 7.78 (dd, J = 9.1, 2.0 Hz, 1H), 7.35-7.28 (m, 4H), 7.23 - 7.17 (m, 5H), 6.88 (d, J = 7.5 Hz, 4H), 5.21 (d, J = 3.3 Hz, 1H), 4.97 (dd, J = 11.2, 3.0 Hz, 1H), 4.86 - 4.75 (m, 1H), 4.52 (s, 1H), 4.47 (d, J = 8.5 Hz, 1H), 4.04 - 3.98 (m, 3H), 3.91 - 3.83 (m, 1H), 3.74 (s, 6H), 3.70 - 3.60 (m, 3H), 3.58 - 3.47 (m, 3H), 3.39 (t, J = 11.7 Hz, 2H), 3.31 (d, J = 4.1 Hz, 1H), 3.19 (qd, J = 8.7, 3.8 Hz, 1H), 3.02 (dt, J = 19.9, 6.8 Hz, 1H), 2.78 - 2.70 (m, 1H), 2.60 (td, J = 5.8, 1.8 Hz, 1H), 2.09 (s, 5H), 1.98 (s, 4H), 1.89 (s, 3H), 1.73 (s, 3H), 1.44 (s, 4H), 1.20 - 0.88 (m, 14H). ³¹P NMR (162 MHz, DMSO-d₆): δ 148.22 (s), 146.82 (t, J = 35.8 Hz).

### II. Preparation of siRNA conjugate

Using solid-phase phosphoramidite method, the synthesis cycle was initiated with compound SA51 prepared in the above step, and nucleoside monomers were linked one by one in the 3'-5' direction according to the nucleotide sequence. The connection of each nucleoside monomer includes four steps: deprotection, coupling, capping, and oxidation or sulfurization. The same synthesis conditions were used for the sense and antisense strands.

Instrument and equipment models: Biolytic Dr. Oligo 48 solid-phase synthesizer, Biocomma Embed CPG Frits universal synthesis column DS0200, Biocomma 96-well plate desalting column DC189650 (80mg). Table 1 shows the reagents used for the synthesis of the siRNA conjugate.

**Table 1**

| Reagent Name | Reagent Composition | Specifi cation | Use | Manufacturer |
|---|---|---|---|---|
| ACT | 0.6M ETT in ACN | 4L | Activator | Kroma |
| Cap A | Acetic anhydride:acetonitrile 2:8 | 4L | Capping reagent | Kroma |
| Cap B | N-methylimidazole:pyridine: acetonit rile 20:30:50 | 4L | | Kroma |
| OXD | 50 nM iodopyridine solution:water 90:10 | 4L | Oxidizing agent | Kroma |
| DCA | Dichloroacetic acid in toluene solution (3% v/v) | 1kg | DMT deprotection reagent | Kroma |
| Acetonitrile | Water content less than 20ppm (actual ~10ppm) | 4L | Solvent | Kroma |
| PADS (Diphenylacetyl disulfide) | Mix 3-methylpyridine and acetonitrile in equal volumes, then prepare 0.2M PADS solution | | Sulfurizing reagent | Kroma |
| 3-Methylpyridine | | | | Kroma |

The synthesis conditions were as follows:
The nucleoside monomer was provided as a 0.05 M solution in acetonitrile. The deprotection reaction conditions for each step were the same, i.e., temperature was 25°C, reaction time was 3 minutes, the deprotection reagent was DCA, and the injection volume was 180 µL.

The coupling reaction conditions for each step were the same, including a temperature of 25°C and a reaction time of 3 minutes. The nucleoside monomer injection volume was 90 µL, and the catalyst ACT injection volume was 110 µL.

The capping conditions for each step were the same, including a temperature of 25°C and a reaction time of 2 minutes. The capping reagent solution was a 1:1 molar ratio mixture of CapA and CapB (CapB1:CapB2=1:1). The capping reagent injection volume was 180 µL.

The oxidation reaction conditions for each step were the same, including a temperature of 25°C, a reaction time of 3 minutes, and an oxidizing agent OXD injection volume of 180 µL.

The sulfurization reaction conditions for each step were the same, including a temperature of 25°C, a reaction time of 4 minutes, and the sulfurizing reagent was 0.05 M PADS in pyridine/acetonitrile solution. The sulfurizing reagent injection volume was 180 µL.

After the last nucleoside monomer was linked, the nucleic acid sequence linked to the solid support was subjected to cleavage, deprotection, purification, and desalting in sequence, followed by lyophilization to obtain the sense strand and the antisense strand, wherein:
Cleavage and deprotection conditions were as follows: a mixture of aqueous ammonia:ethanol = 3:1 was added to the synthesized nucleotide sequence linked to the support to a volume of 0.8 mL. The reaction was carried out at 50°C for 15 h. The remaining support was removed by filtration, and the supernatant was concentrated to dryness under vacuum.
Purification and desalting conditions were as follows: a C18 reverse-phase chromatography column was used for desalting. Specific conditions included:
   (1) Sample preparation
      0.1 M TEAA (triethylammonium acetate) was added to the oligonucleotide sample to a volume of 0.8 mL.
   (2) Activation of the 96-well plate
      Activation: 0.8 mL of acetonitrile was passed through each well of the 96-well plate for activation;
      Equilibration: The 96-well plate was equilibrated with 0.8 mL of TEAA (pH 7.0) solution.
   (3) The purification process was performed sequentially as follows:
      0.8 mL of the solution containing the oligonucleotide was passed through the desalting column;
      The 96-well plate was washed twice with 0.8 mL of 6.5% aqueous ammonia to remove failed sequences;
      The 96-well plate was rinsed twice with 0.8 mL of deionized water to remove salts;
      The 96-well plate was rinsed 3 times with 0.8 mL of 3% trifluoroacetic acid to remove DMT, and the adsorption layer was observed to turn orange-red;
      The 96-well plate was rinsed with 0.8 mL of 0.1 M TEAA;
      The 96-well plate was rinsed twice with 0.8 mL of deionized water to remove trifluoroacetic acid and residual salts;
      Elution was performed with 0.6 mL of 20% acetonitrile, and the eluate was collected and lyophilized.

The detection method was as follows: a Waters Acquity UPLC-LTQ LCMS (column: ACQUITY UPLC BEH C18) was used to detect the purity of the above sense and antisense strands and analyze their molecular weights. The measured values were consistent with the theoretical values, indicating that the synthesized products were the sense strand and antisense strand with groups conjugated at the 3' end and/or 5' end.

The annealing operation was as follows: the sense and antisense strands obtained in step 2 were dissolved separately in water for injection to prepare solutions of 0.1 mg/mL-40 mg/mL. The concentrations were determined with a concentration meter, and they were mixed in an equimolar ratio, heated at 90°C for 5 minutes, and then slowly cooled naturally to allow them to form a double-stranded structure through hydrogen bonds. A sample was taken to detect the SEC purity of the product. The double-stranded sample was lyophilized.

### Example 3 Synthesis of Ago, Cgo, Ugo monomers and preparation of siRNA containing this modification

3.1. The synthesis route of the Ago monomer is described in Example 2 of the applicant's patent application No. 202310436173.7, and the specific synthesis method is as follows: 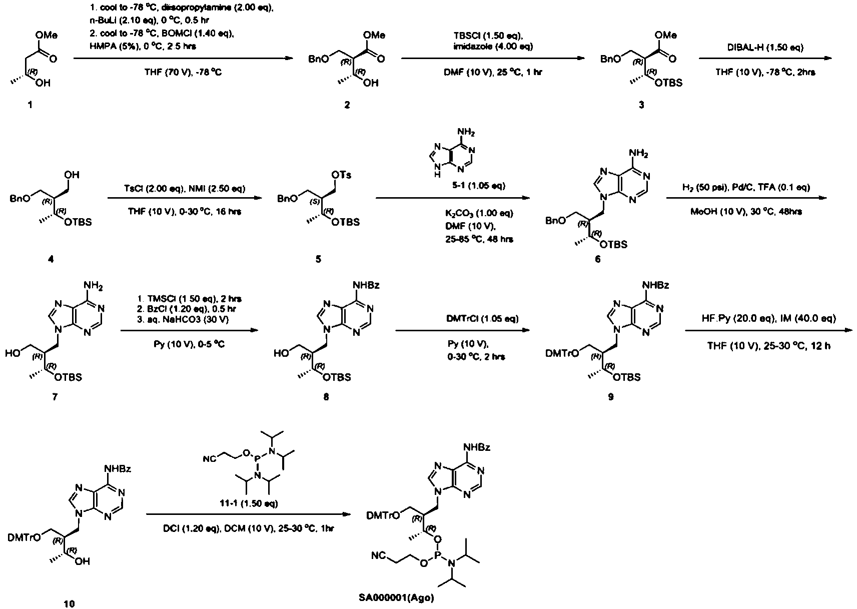
(1) Anhydrous diisopropylamine (16.2 g, 160.0 mmol, 22.6 mL, 2.0 equiv) was dissolved in 350 mL of anhydrous tetrahydrofuran, cooled to between -70°C and -78°C, and protected by nitrogen displacement. n-Butyllithium solution (2.5 M, 67.1 mL) was added dropwise (slowly, dropwise addition for at least 10 minutes), and the reaction solution continued to stir at -70°C to - 78°C for half an hour. Compound 1 (9.44 g, 79.9 mmol, 9.17 mL, 1.0 equiv) was dissolved in 175 mL of anhydrous tetrahydrofuran, cooled to between -70°C and -78°C, and under nitrogen protection, the solution obtained in the previous step was added dropwise (slowly, dropwise addition for at least 10 minutes). The reaction solution continued to stir at -70°C to -78°C for half an hour. While maintaining the previous temperature, hexamethylphosphoramide (26.1 g, 146 mmol, 25.6 mL, 1.82 equiv) and benzyl chloromethyl ether (17.5 g, 112 mmol, 15.5 mL, 1.4 equiv) were slowly added dropwise to the previous reaction solution (slowly, dropwise addition for at least 10 minutes). After the addition was complete, the temperature was raised to 0°C and stirring was continued for 3 hours. TLC and LCMS detected the disappearance of compound 1. The reaction was quenched by adding 600 mL of saturated ammonium chloride solution in two batches. The mixture was extracted with 200 mL of methyl tert-butyl ether. The organic phase was collected, washed with saturated brine, dried over anhydrous sodium sulfate, filtered, and the filtrate was concentrated. The resulting crude product was purified by silica gel column chromatography (petroleum ether/ethyl acetate = 100/1 to 1/1) to obtain compound 2 as a brownish substance (9.69 g, 40.7 mmol, 51% yield). ¹H NMR (400 MHz, CDCl₃): δ 7.46-7.28 (m, 10H), 4.58-4.46 (m, 2H), 4.19-4.08 (m, 1H), 3.82-3.67 (m, 5H), 2.77 (q, J = 6.1 Hz, 1H), 1.24 (d, J = 6.5 Hz, 4H). LC-MS: C₁₃H₁₈O₄, molecular weight 238.1, 239.1 (M+H).
(2) Compound 2 (14.7 g, 61.7 mmol, 1.0 equiv) was dissolved in 150 mL of dichloromethane. Under nitrogen protection at room temperature, imidazole (16.8 g, 247.0 mmol, 4.0 equiv) and tert-butyldimethylsilyl chloride (27.9 g, 185.0 mmol, 22.7 mL, 3.0 equiv) were added. The reaction solution was stirred at room temperature for one hour. TLC and LCMS showed the disappearance of compound 2. 100 mL of dichloromethane was added to the reaction solution, and the reaction solution was washed twice with 400 mL of saturated brine. The organic phase was dried and concentrated after filtration. The resulting crude product was purified by silica gel column chromatography (petroleum ether/ethyl acetate = 100/1 to 10/1) to obtain compound 3 as a pale yellow oil (12.0 g, 34.0 mmol, 55% yield). ¹H NMR (400 MHz, CDCl₃): δ 7.40-7.25 (m, 5H), 4.52 (d, J = 2.4 Hz, 2H), 4.10 (t, J = 6.3 Hz, 1H), 3.75-3.65 (m, 4H), 3.59 (dd, J = 9.2, 5.3 Hz, 1H), 2.79 (dt, J = 8.4, 5.9 Hz, 1H), 1.17 (d, J = 6.2 Hz, 3H), 0.86 (s, 9H), 0.04 (d, J = 8.6 Hz, 6H). LC-MS: C₁₉H₃₂O₄Si, molecular weight 352.1, 353.2 (M+H).
(3) Compound 3 (11.1 g, 31.5 mmol, 1.0 equiv) was dissolved in tetrahydrofuran, cooled to between -70°C and -60°C, and under nitrogen protection, diisobutylaluminium hydride (1.0 M, 69.3 mL, 2.2 equiv) was added dropwise. Stirring was continued at this temperature for two hours. LCMS showed the disappearance of compound 3. The temperature was raised to 0°C, 20 mL of ethyl acetate was added, and the reaction was quenched with 100 mL of potassium sodium tartrate solution, followed by stirring for half an hour. The mixture was washed with 100 mL of saturated brine, dried over anhydrous sodium sulfate, filtered, and concentrated to obtain compound 4 as a pale yellow oil (9.75 g, 30.0 mmol, 95% yield). ¹H NMR (400 MHz, CDCl₃): δ 7.40-7.27 (m, 5H), 4.59-4.45 (m, 2H), 4.19 (dd, J = 6.2, 3.7 Hz, 1H), 4.01 (dd, J = 11.3, 4.0 Hz, 1H), 3.78-3.60 (m, 3H), 1.78-1.68 (m, 1H), 1.29-1.21 (m, 3H), 0.95-0.87 (m, 9H), 0.12-0.02 (m, 6H). LC-MS: C₁₈H₃₂O₃Si, molecular weight 324.1, 325.2 (M+H).
(4) Compound 4 (9.75 g, 30.0 mmol, 1.0 equiv) was dissolved in tetrahydrofuran, cooled to 0°C, and under nitrogen protection, p-toluenesulfonyl chloride (11.5 g, 60.1 mmol, 2.0 equiv) and methylimidazole (6.17 g, 75.1 mmol, 5.99 mL, 2.5 equiv) were added dropwise. After the addition was complete, the temperature was raised to room temperature and stirring was continued for 16 hours. LCMS showed the disappearance of compound 4. 20 mL of ethyl acetate was added to the reaction solution, and under an ice bath, the reaction was quenched with 100 mL of potassium sodium tartrate solution, and stirring was continued for half an hour. The mixture was washed with 100 mL of saturated brine, dried over anhydrous sodium sulfate, filtered, and concentrated to obtain the crude product, compound 5, as a pale yellow oil (13.7 g, 28.6 mmol, 95% yield). ¹H NMR (400 MHz, CDCl₃): δ 7.89-7.68 (m, 2H), 7.42-7.17 (m, 10H), 4.47-4.33 (m, 2H), 4.23 (dd, J = 9.6, 5.1 Hz, 1H), 4.13-4.06 (m, 1H), 4.01-3.94 (m, 1H), 3.52-3.34 (m, 2H), 2.43 (s, 3H), 2.02-1.89 (m, 1H), 1.10 (d, J = 6.3 Hz, 3H), 0.81 (s, 8H), 0.04--0.05 (m, 6H). LC-MS: C₂₅H₃₈O₅SSi, molecular weight 478.1, 479.2 (M+H).
(5) Under nitrogen protection, compound 5 (13.7 g, 28.6 mmol, 1.0 equiv) and 35 mL of acetonitrile were added to a dry reaction flask. The acetonitrile was removed by distillation at 35-40°C to remove water from compound 5. Under nitrogen protection, 80 mL of N,N-dimethylformamide, compound 5-1 (9H-purin-6-amine) (4.25 g, 31.5 mmol, 1.1 equiv), and potassium carbonate (3.96 g, 28.6 mmol, 1.0 equiv) were added to another clean and dry reaction flask. The temperature was raised to 95-100°C and stirring was continued for half an hour. At this temperature, a solution of compound 5 (13.7 g, 28.6 mmol, 1.0 equiv) in N,N-dimethylformamide (60 mL) was added dropwise to the reaction solution, and stirring was continued for 12 hours. LCMS showed the disappearance of compound 5. The reaction solution was cooled to room temperature, and 200 mL of ethyl acetate was added. The mixed solution was washed sequentially with 200 mL of sodium bicarbonate solution and 100 mL of saturated brine. The organic phase was dried over anhydrous sodium sulfate, filtered, and the filtrate was concentrated. The resulting crude product was purified by silica gel column chromatography (petroleum ether/ethyl acetate = 20/1 to 10/1) to obtain compound 6 as a pale yellow oil (6.2 g, 14.0 mmol, 49% yield). ¹H NMR(400 MHz, CDCl₃): δ 8.37 (s, 1H), 7.75 (s, 1H), 7.39-7.24 (m, 6H), 5.66 (br, s, 2H), 4.47-4.34 (m, 3H), 4.21 (dd, J = 14.1, 8.8 Hz, 1H), 4.07 (dd, J = 6.2, 4.9 Hz, 1H), 3.42-3.34 (m, 2H), 2.10-1.96 (m, 1H), 1.19 (d, J = 6.4 Hz, 3H), 0.90 (s, 9H), 0.06 (d, J = 6.0 Hz, 6H). LC-MS: C₂₃H₃₅N₅O₂Si, molecular weight 441.1, 442.3 (M+H).
(6) At room temperature, palladium on carbon (3.0 g, 10% purity) was dissolved in 25 mL of methanol. 25 mL of a methanol solution of compound 6 (5.2 g, 11.8 mmol, 1.0 equiv) and trifluoroacetic acid (134.0 mg, 1.18 mmol, 87.2 uL) were added. The reaction solution was stirred under a hydrogen pressure of 50 psi for 48 hours. LCMS showed the disappearance of compound 6. The reaction solution was filtered and concentrated to obtain the crude product, compound 7, as a pale yellow oil (3.9 g, 11.1 mmol). ¹H NMR (400 MHz, CDCl₃): δ 8.21 (s, 1H), 8.12 (s, 1H), 4.50-4.36 (m, 1H), 4.33-4.22 (m, 1H), 4.15-4.02 (m, 1H), 3.53 (d, J = 5.9 Hz, 2H), 2.21-2.09 (m, 1H), 1.25 (d, J = 6.4 Hz, 3H), 0.88 (s, 9H), 0.05 (d, J = 3.1 Hz, 6H). LC-MS: C₁₆H₂₉N₅O₂Si, molecular weight 351.1, 352.2 (M+1).
(7) Compound 7 (3.9 g, 11.1 mmol, 1.0 equiv) was placed in a clean and dry reaction flask. Under nitrogen protection, 10 mL of pyridine was added, and the temperature was raised to distill off the pyridine. This operation was repeated once to remove water from compound 7. 28 mL of pyridine was added, and under an ice bath, trimethylsilyl chloride (4.8 g, 44.4 mmol, 5.63 mL, 4.0 equiv) was added. The temperature was raised to room temperature, and stirring was continued for two hours. TLC showed the disappearance of compound 7. Under an ice bath, benzoyl chloride (6.2 g, 44.4 mmol, 5.15 mL, 4.0 equiv) was added, and stirring was continued for 2 hours. TLC showed the disappearance of the starting material. 60 mL of water and 90 mL of concentrated ammonia water were added dropwise, and stirring was continued for half an hour. The reaction solution was extracted with 250 mL of ethyl acetate. The organic phase was washed with saturated brine, dried, filtered, and concentrated. The resulting crude product was purified by silica gel column chromatography (elution system: dichloromethane/methanol = 50/1 to 10/1) to obtain compound 8 as a pale yellow oil (4.0 g, 8.78 mmol, 79% yield). ¹H NMR (400 MHz, CDCl₃): δ 9.22-8.90 (m, 1H), 8.79 (s, 1H), 8.07-8.04 (m, 2H), 8.03 (d, J = 1.4 Hz, 1H), 7.65-7.59 (m, 1H), 7.57-7.50 (m, 2H), 4.62-4.50 (m, 1H), 4.42 (dd, J = 14.3, 8.6 Hz, 1H), 4.24-4.09 (m, 1H), 4.05 (d, J = 6.3 Hz, 1H), 3.59-3.48 (m, 1H), 3.47-3.35 (m, 1H), 2.04-1.98 (m, 1H), 1.31 (d, J = 6.3 Hz, 3H), 0.98-0.84 (m, 9H), 0.11 (d, J = 10.1 Hz, 5H). LC-MS: C₂₃H₃₃N₅O₃Si, molecular weight 455.2, 456.3 (M+H).
(8) Compound 8 (4.0 g, 8.78 mmol, 1.0 equiv) was placed in a clean and dry reaction flask. Under nitrogen protection, 10 mL of pyridine was added, and the temperature was raised to distill off the pyridine. This operation was repeated once to remove water from compound 8. 28 mL of pyridine was added, and under an ice bath, 4,4'-dimethoxytrityl chloride (3.27 g, 9.66 mmol, 1.1 equiv) was added. The temperature was raised to room temperature and stirring was continued for 1 hour. TLC showed the disappearance of compound 8. 150 mL of ethyl acetate was added to the reaction mixture, and it was washed with 200 mL of sodium bicarbonate solution and 150 mL of saturated brine. The organic phase was dried, filtered, and the filtrate was concentrated. The resulting crude product was purified by silica gel column chromatography (gradient elution: petroleum ether/ethyl acetate = 10/1 to 0/1) to obtain compound 9 as a pale yellow oil (5.91 g, 7.8 mmol, 88% yield). ¹H NMR (400 MHz, CDCl₃): δ 9.07 (br s, 1H), 8.82 (s, 1H), 8.04 (d, J = 7.1 Hz, 2H), 7.80 (s, 1H), 7.65-7.58 (m, 1H), 7.57-7.50 (m, 2H), 7.28-7.13 (m, 9H), 6.81-6.69 (m, 4H), 4.52-4.43 (m, 1H), 4.25 (dd, J = 14.2, 8.6 Hz, 1H), 4.07 (dd, J = 6.3, 4.2 Hz, 1H), 3.76 (d, J = 4.5 Hz, 6H), 3.22 (dd, J = 9.8, 5.4 Hz, 1H), 3.04 (dd, J = 9.8, 6.1 Hz, 1H), 2.34 (dd, J = 8.1, 4.5 Hz, 1H), 1.19 (d, J = 6.3 Hz, 3H), 0.90-0.77 (m, 9H), 0.07--0.09 (m, 6H). LC-MS: C₄₄H₅₁N₅O₅Si, molecular weight 757.3, 758.4 (M+H).
(9) Compound 9 (3.00 g, 3.96 mmol, 1.0 equiv) was dissolved in tetrahydrofuran. Under nitrogen protection and in an ice bath, hydrogen fluoride-pyridine salt (2.26 g, 79.2 mmol, 2.1 mL, 20.0 equiv) and imidazole (10.8 g, 158.0 mmol, 40.0 equiv) were added. The temperature was raised to room temperature, and stirring was continued for 2 hours. LCMS showed the disappearance of compound 9. 50 mL of ethyl acetate was added, and it was washed with 100 mL of sodium bicarbonate solution and 50 mL of saturated brine. After drying and filtration, the filtrate was concentrated. The resulting crude product was purified by silica gel column chromatography (gradient elution: petroleum ether/ethyl acetate = 10/1 to 0/1) to obtain compound 10 as a pale yellow oil (2.44 g, 3.79 mmol, 96% yield). ¹H NMR (400 MHz, DMSO-d₆): δ 11.16 (br s, 1H), 8.70 (s, 1H), 8.33 (s, 1H), 8.05 (d, J = 7.4 Hz, 2H), 7.69-7.61 (m, 1H), 7.60-7.52 (m, 2H), 7.25-7.12 (m, 5H), 7.05 (t, J = 8.9 Hz, 4H), 6.78 (dd, J = 8.9, 3.1 Hz, 4H), 4.79 (d, J = 4.0 Hz, 1H), 4.51-4.28 (m, 2H), 3.81 (d, J = 4.0 Hz, 1H), 3.70 (s, 6H), 3.10 (dd, J = 9.8, 4.9 Hz, 1H), 2.89 (dd, J = 9.7, 5.1 Hz, 1H), 2.37-2.23 (m, 1H), 1.03 (d, J = 6.3 Hz, 3H). LC-MS: C₃₈H₃₇N₅O₅, molecular weight 643.2, 644.2 (M+H).
(10) Compound 10 (1.5 g, 2.33 mmol, 1.0 equiv) was placed in a clean and dry reaction flask. Under nitrogen protection, 4 mL of acetonitrile was added, and the temperature was raised to 35-40°C to distill off the acetonitrile. This operation was repeated once to remove water from compound 10. At room temperature, 15 mL of anhydrous dichloromethane was added to the above reaction flask. Then, compound 11-1 (1.05 g, 3.5 mmol, 1.11 mL, 1.5 equiv) and 4,5-dicyanoimidazole (358 mg, 3.03 mmol, 1.3 equiv) were added, and stirring was continued at room temperature for one hour. TLC showed the disappearance of compound 10. 30 mL of dichloromethane was added, and it was washed with sodium bicarbonate solution (50 mL x 2) and saturated brine (50 mL). The organic phase was dried, filtered, and the filtrate was concentrated.
The resulting crude product was dissolved in 50 mL of methyl tert-butyl ether, and 100 mL of 1% aqueous sodium hydroxide solution was added, followed by stirring for half an hour. The layers were allowed to separate and the organic phase was collected. The organic phase was washed with saturated brine, dried, filtered, and concentrated. The crude product was purified by C18 reverse-phase column chromatography to obtain product SA000001 as a pale yellow substance (1.1 g, 1.3 mmol, 56% yield). ¹H NMR (400 MHz, CD₃CN): δ 9.33 (br s, 1H), 8.61 (br s, 1H), 7.99 (d, J = 11.0 Hz, 3H), 7.67-7.60 (m, 1H), 7.59-7.49 (m, 2H), 7.32-6.99 (m, 9H), 6.75 (td, J = 9.2, 6.5 Hz, 4H), 4.50-4.38 (m, 1H), 4.36-4.13 (m, 2H), 3.72 (dd, J = 4.8, 1.5 Hz, 6H), 3.66-3.45 (m, 3H), 3.25 (td, J = 9.6, 5.4 Hz, 1H), 3.01 (ddd, J = 17.9, 10.0, 5.8 Hz, 1H), 2.62 (t, J = 5.9 Hz, 1H), 2.53 (t, J = 5.9 Hz, 1H), 1.32-1.22 (m, 4H), 1.17-1.05 (m, 12H). ³¹P NMR (DMSO-d₆, 162 MHz): δ ppm 147.7, 146.8. LCMS: C₄₇H₅₄N₇O₆P, molecular weight 843.3, 844.5 (M+H).
3.2. The synthesis route of the Cgo monomer is described in Example 4 of the applicant's patent application No. 202310436173.7, and the specific synthesis method is as follows: 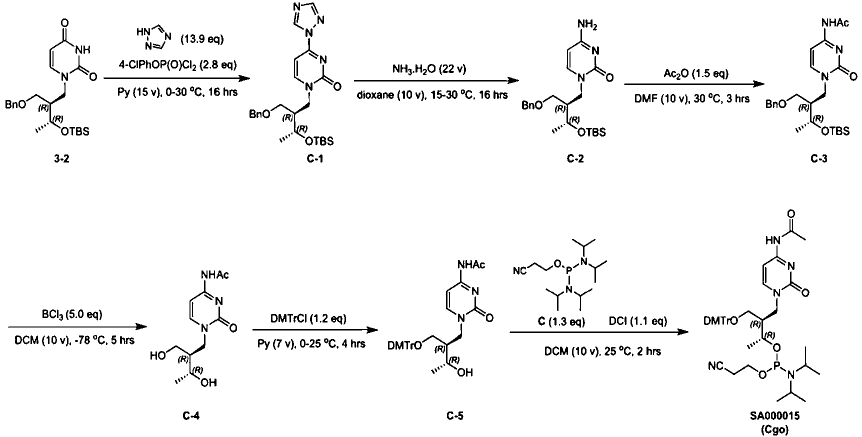
(1) At room temperature, compound 3-2 (8.5 g, 20.3 mmol, prepared in Example 5) and 1,2,4-triazole (19.5 g, 282.0 mmol, 13.9 equiv) were dissolved in pyridine (128.0 mL). Then, under an ice-water bath, 4-chlorophenyl dichlorophosphate (56.9 mmol, 9.25 mL, 2.8 equiv) was added dropwise to the reaction system. The reaction solution was stirred at 30°C for 16 hours. After the reaction was complete, the solvent pyridine was removed by distillation under reduced pressure. The remaining system was extracted 3 times with water and ethyl acetate. The organic phases were combined and concentrated by rotary evaporation to obtain the crude product, compound C-1, as a yellow oil (9.5 g), which was used directly in the next step. Mass spectrum identification of compound C-1 (C₂₄H₃₅N₅O₃Si, molecular weight 469.1, [M+H] = 470.3).
(2) At room temperature, compound C-1 (9.5 g, 20.2 mmol) was dissolved in 1,4-dioxane (95.0 mL). Then, aqueous ammonia (1.38 mol, 212.1 mL, 25% purity, 68.0 equiv) was added to the reaction system. The reaction solution was stirred at 30°C for 16 hours. After the reaction was complete, the solvent was removed by distillation under reduced pressure. The remaining system was extracted 3 times with water and dichloromethane. The organic phases were combined and concentrated by rotary evaporation to obtain the crude product, compound C-2, as a yellow solid (8.5 g), which was used directly in the next step. Mass spectrum identification of compound C-2 (C₂₂H₃₅N₃O₃Si, molecular weight 417.1, [M+H] = 418.2).
(3) At room temperature, compound C-2 (8.5 g, 20.2 mmol) was dissolved in N,N-dimethylformamide (85.0 mL). Then, acetic anhydride (3.1 g, 30.4 mmol, 2.85 mL, 1.5 equiv) was slowly added to the reaction system. The reaction solution was stirred at 30°C for 3 hours. After the reaction was complete, the solvent was removed by distillation under reduced pressure. The remaining system was extracted 3 times with saturated sodium bicarbonate solution and ethyl acetate. The organic phases were combined and concentrated by rotary evaporation. The resulting crude product was purified by silica gel column chromatography (dichloromethane/methanol 1:0 to 100:1) to obtain compound C-3 as a yellow solid (4.2 g, reaction yield 43%). ¹H NMR data of compound C-3: (400 MHz, DMSO-d₆) δ 10.7 (s, 1H), 7.79 (d, J = 7.2 Hz, 1H), 7.29 (m, 5H), 7.08 (d, J = 7.2 Hz, 1H), 4.36 (d, J = 1.2 Hz, 2H), 4.05-3.99 (m, 2H), 3.72-3.67 (m, 1H), 3.39 (dd, J = 6.4 Hz, 2H), 2.15-2.08 (m, 1H), 2.08 (s, 3H), 1.15 (d, J = 6.4 Hz, 3H), 0.85 (s, 9H), 0.02 (d, J = 6.8 Hz, 6H). Mass spectrum identification of compound C-3 (C₂₄H₃₇N₃O₄Si, molecular weight 459.1, [M+H] = 460.2).
(4) At -78°C, compound C-3 (4.3 g, 9.35 mmol) was dissolved in dichloromethane (46.0 mL). Then, boron trichloride (1.0 M in DCM, 46.8 mL, 5.0 equiv) was slowly added to the reaction system. The reaction solution was then stirred at -78°C for 4 hours. After the reaction was complete, the reaction was quenched with triethylamine (40.0 mL) and methanol (88.0 mL). The remaining system was extracted 3 times with water and dichloromethane. The organic phases were combined and concentrated by rotary evaporation. The resulting crude product was purified by C18 reverse-phase column chromatography to obtain compound C-4 as a white solid (0.96 g, reaction yield 40%). ¹H NMR data of compound C-4: (400 MHz, DMSO-d₆) δ 10.7 (s, 1H), 8.01 (d, J = 7.2 Hz, 1H), 7.12 (d, J = 7.2 Hz, 1H), 4.60 (d, J = 4.8 Hz, 2H), 4.53-4.50 (m, 1H), 4.00-3.99 (m, 1H), 3.75-3.70 (m, 2H), 3.37-3.32 (m, 2H), 2.08 (s, 3H), 1.81-1.77 (m, 1H), 1.01 (d, J = 6.4 Hz, 3H). Mass spectrum identification of compound C-4 (C₁₁H₁₇N₃O₄, molecular weight 255.1, [M+H] = 256.2).
(5) At room temperature, compound C-4 (1.5 g, 5.88 mmol) was dissolved in pyridine (10.0 mL). Under an ice-water bath, 4,4'-dimethoxytrityl chloride (2.4 g, 7.05 mmol, 1.2 equiv) was added to the reaction system. The reaction solution was then stirred at room temperature for 4 hours. After the reaction was complete, the solvent pyridine was removed by distillation under reduced pressure. The remaining system was extracted 3 times with saturated sodium bicarbonate solution and ethyl acetate. The organic phases were combined and concentrated by rotary evaporation. The resulting crude product was purified by silica gel column chromatography (petroleum ether/ethyl acetate 4:1 to 0:1) to obtain compound C-5 as a white solid (0.7 g, reaction yield 70%). ¹H NMR data of compound C-5: (400 MHz, CDCl₃) δ 9.71 (s, 1H), 7.39(d, J = 7.3 Hz, 3H), 7.35-7.21 (m, 7H), 7.06 (d, J = 7.1 Hz, 1H), 6.85 (d, J = 8.8 Hz, 4H), 4.71 (d, J = 3.3 Hz, 1H), 4.43 (dd, J = 13.8, 4.5 Hz, 1H), 4.23-3.99 (m, 2H), 3.80 (s, 6H), 3.50-3.38 (m, 1H), 3.31 (dd, J = 10.0, 4.1 Hz, 1H), 2.69 (t, J = 9.4 Hz, 1H), 2.21 (s, 3H), 1.08 (d, J = 6.1 Hz, 3H). Mass spectrum identification of compound C-5 (C₃₂H₃₅N₃O₆, molecular weight 557.1, [M+H] = 558.4).
(6) At room temperature under nitrogen, compound C-5 (2.0 g, 3.59 mmol) was dissolved in dichloromethane (20.0 mL). 4,5-dicyanoimidazole (466.0 mg, 3.95 mmol, 1.1 equiv) and bis(diisopropylamino)(2-cyanoethoxy)phosphine (1.4 g, 4.66 mmol, 1.48 mL, 1.3 equiv) were added to the reaction system, respectively. The reaction solution was then stirred at room temperature for 2 hours. After the reaction was complete, the reaction solution was dissolved in methyl tert-butyl ether (10.0 mL) and 1% sodium hydroxide solution (10.0 mL) and stirring was continued at room temperature for 0.5 hours. The remaining system was extracted 3 times with saturated sodium bicarbonate solution and dichloromethane. The organic phases were combined and concentrated by rotary evaporation. The resulting crude product was purified by C18 reverse-phase column chromatography to obtain compound SA000015 as a white solid (1.5 g, reaction yield 55%). ¹H NMR data of compound SA000015: (400 MHz, CD₃CN) δ 9.15 (s, 1H), 7.50-7.18 (m, 11H), 6.84-6.80 (m, 4H), 4.22-4.08 (m, 2H), 3.75-3.22 (m, 11H), 3.23-3.22 (m, 2H), 2.62-2.51 (m, 2H), 2.38-2.35 (m, 1H), 2.12 (s, 3H), 1.022-1.07 (m, 15H). ³¹P NMR data: (400 MHz, CD₃CN) δ 147.5, 146.7. Mass spectrum identification of compound SA000015 (C₄₁H₅₂N₅O₇P, molecular weight 757.1, [M+H] = 758.5).
3.3. The synthesis route of the Ugo monomer is described in Example 5 of the applicant's patent application No. 202310436173.7, and the specific synthesis method is as follows: 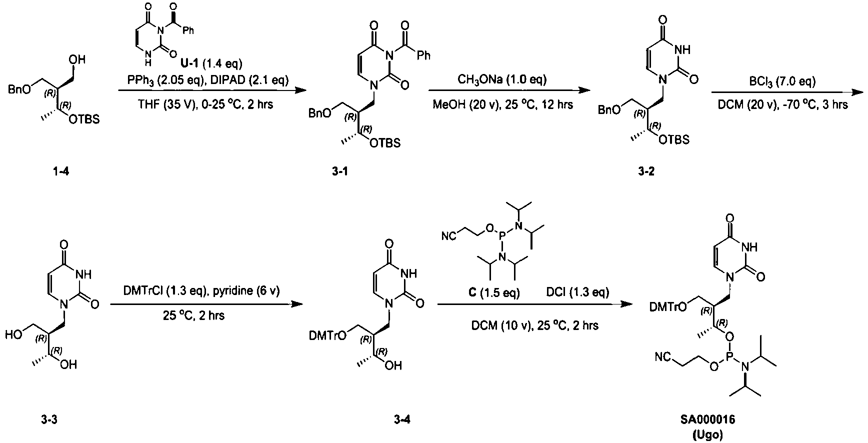
(1) Under an ice-water bath, compound 1-4 (9.99 g, 30.8 mmol), compound U-1 (commercially available, purchased from Shanghai Haohong Biomedical Technology Co., Ltd.) (9.33 g, 43.1 mmol, 1.4 equiv), and triphenylphosphine (16.6 g, 63.2 mmol, 2.0 equiv) were dissolved in dry tetrahydrofuran (350.0 mL), respectively. Then, diisopropyl azodicarboxylate (13.1 g, 64.7 mmol, 12.6 mL, 2.1 equiv) was added dropwise to the reaction system. The reaction solution was then stirred at room temperature for 2 hours. After the reaction was complete, the reaction solution was extracted 3 times with saturated sodium bicarbonate solution and ethyl acetate. The organic phases were combined and concentrated by rotary evaporation. The resulting crude product was purified by silica gel column chromatography (petroleum ether/ethyl acetate 10: 1 to 8:1) to obtain compound 3-1 as a white solid (16.1 g, reaction yield 99%). ¹H NMR data of compound 3-1: (400 MHz, DMSO-d₆) δ 7.89-7.74 (m, 4H), 7.56-7.53 (m, 2H), 7.33-7.29 (m, 5H), 5.80-5.73 (m, 1H), 4.46-4.38 (m, 2H), 4.00-3.91 (m, 2H), 3.60-3.50 (m, 1H), 3.49-3.43 (m, 2H) 2.17-1.98 (m, 1H), 1.13 (s, 3H), 0.83 (s, 9H), 0.01 (d, J = 6.0 Hz, 6H). Mass spectrum identification of compound 3-1 (C₂₉H₃₈N₂O₅Si, molecular weight 522.1, [M+H] = 523.2).
(2) At room temperature, compound 3-1 (8.05 g, 15.4 mmol) was dissolved in methanol (160.0 mL). Then, sodium methoxide (2.77 g, 15.4 mmol, 30% purity, 1.0 equiv) was slowly added to the reaction system. The reaction solution was then stirred at room temperature for 12 hours. After the reaction was complete, the solvent methanol was removed by distillation under reduced pressure. The remaining system was extracted 3 times with 1M hydrochloric acid solution and ethyl acetate. The organic phases were combined and concentrated by rotary evaporation. The resulting crude product was purified by silica gel column chromatography (petroleum ether/ethyl acetate 10:1 to 0:1) to obtain compound 3-2 as a white solid (6.1 g, reaction yield 94%). ¹H NMR data of compound 3-2: (400 MHz, DMSO-d₆) δ 11.1 (s, 1H), 7.48 (d, J = 8.0 Hz, 1H), 7.30-7.24 (m, 5H), 5.47-5.44 (m, 1H), 4.45-4.35 (m, 2H), 3.98-3.94 (m, 1H), 3.79-3.78 (m, 1H), 3.63-3.57 (m, 1H), 3.38-3.35 (m, 2H), 2.04-1.95 (m, 1H), 1.10 (s, 3H), 0.83 (s, 9H), 0.01 (d, J = 6.0 Hz, 6H). Mass spectrum identification of compound 3-2 (C₂₂H₃₄N₂O₄Si, molecular weight 418.1, [M+H] = 418.2).
(3) At -70°C, compound 3-2 (4.0 g, 9.56 mmol) was dissolved in dichloromethane (30.0 mL). Then, boron trichloride (1.0 M in DCM, 66.9 mL, 7.0 equiv) was slowly added to the reaction system. The reaction solution was then stirred at -70°C for 3 hours. After the reaction was complete, the reaction was quenched with triethylamine (5.0 mL) and methanol (30.0 mL). The remaining system was extracted 3 times with water and dichloromethane. The organic phases were combined and concentrated by rotary evaporation. The resulting crude product was purified by C18 reverse-phase column chromatography to obtain compound 3-3 as a white solid (0.7 g, reaction yield 33%). ¹H NMR data of compound 3-3: (400 MHz, DMSO-d₆) δ 7.54 (d, J = 8.0 Hz, 1H), 5.51 (d, J = 7.6 Hz, 1H), 3.88-3.84 (m, 1H), 3.72-3.70 (m, 1H), 3.61-3.59 (m, 2H), 1.75-1.68 (m, 1H), 1.09 (d, J = 6.4 Hz, 3H). Mass spectrum identification of compound 3-3 (C₉H₁₄N₂O₄, molecular weight 214.1, [ M+H] = 215.2).
(4) At room temperature, compound 3-1 (0.4 g, 1.87 mmol) was dissolved in pyridine (4.0 mL). Under an ice-water bath, 4,4'-dimethoxytrityl chloride (949.0 mg, 2.8 mmol, 1.5 equiv) was added to the reaction system. The reaction solution was then stirred at room temperature for 2 hours. After the reaction was complete, the solvent pyridine was removed by distillation under reduced pressure. The remaining system was extracted 3 times with saturated sodium bicarbonate solution and ethyl acetate. The organic phases were combined and concentrated by rotary evaporation. The resulting crude product was purified by silica gel column chromatography (petroleum ether/ethyl acetate 4:1 to 0:1) to obtain compound 3-4 as a white solid (0.7 g, reaction yield 70%). ¹H NMR data of compound 3-4: (400 MHz, DMSO-d₆) δ 11.3 (s, 1H), 7.37 (d, J = 8.0 Hz, 1H), 7.33-7.29 (m, 4H), 7.20-7.17 (m, 5H), 6.86-6.83 (m, 4H), 5.40 (d, J = 8.0 Hz, 1H), 4.61 (d, J = 4.8 Hz, 2H), 3.83-3.79 (m, 2H), 3.72 (s, 6H), 3.71-3.68 (m, 1H), 3.08-2.90 (m, 2H), 1.90 (s, 1H), 1.01 (d, J = 6.4 Hz, 3H). Mass spectrum identification of compound 3-4 (C₃₀H₃₂N₂O₆, molecular weight 516.1, [M+H] = 517.4).
(5) At room temperature under nitrogen, compound 3-4 (1.2 g, 2.32 mmol) was dissolved in dichloromethane (12.0 mL). 4,5-dicyanoimidazole (357.0 mg, 3.02 mmol, 1.3 equiv) and bis(diisopropylamino)(2-cyanoethoxy)phosphine (1.05 g, 3.48 mmol, 1.5 equiv) were added to the reaction system, respectively. The reaction solution was then stirred at room temperature for 2 hours. After the reaction was complete, the reaction solution was dissolved in methyl tert-butyl ether (10.0 mL) and 1% sodium hydroxide solution (10.0 mL) and stirring was continued at room temperature for 0.5 hours. The remaining system was extracted 3 times with ethyl acetate. The organic phases were combined and concentrated by rotary evaporation. The resulting crude product was purified by C18 reverse-phase column chromatography to obtain compound SA000016 as a white solid (1.2 g, reaction yield 71%). ¹H NMR data of compound SA000016: (400 MHz, CD₃CN) δ 9.39 (s, 1H), 7.39-7.24 (m, 10H), 6.85-6.81 (m, 4H), 5.43-5.39 (m, 1H), 4.21-4.18 (m, 1H), 3.86-3.77 (m, 1H), 3.75 (s, 6H), 3.56-3.53 (m, 5H), 3.28-3.05 (m, 2H), 2.62-2.61 (m, 2H), 2.22-2.19 (m, 2H), 1.22-1.07 (m, 15H). ³¹P NMR data: (400 MHz, CD₃CN) δ 147.6, 146.5. Mass spectrum identification of compound SA000016 (C₃₉H₄₉N₄O₇P, molecular weight 716.1, [M+H] = 717.5).

### 3.4 Preparation of modified siRNA

The nucleotide at position 6 or 7 of the 5' region of the antisense strand contains a chemical modification as shown by Ago, Cgo, or Ugo. The modified siRNA was obtained by solid-phase synthesis as described in Example 2.

The inventors designed and synthesized a large number of PD-L1 siRNAs through preliminary work. After verification of cell activity, some PD-L1 siRNAs with poor effects were excluded, and some PD-L1 siRNAs with better activity were retained for further experiments. The specific sequences are shown in Tables 2 to 4 below.

Table 2 below shows the unmodified sense and antisense strands of the PD-L1 siRNAs. Table 3 below shows the modified sense and antisense strands of the PD-L1 siRNAs (this table lists the modified sequences corresponding to each siRNA in Table 1 from top to bottom). Table 4 shows the siRNA conjugate sequences. In the tables, the capital letters A, C, G, and U represent adenosine-3'-phosphate, cytidine-3'-phosphate, guanosine-3'-phosphate, and uridine-3'-phosphate, respectively; the lowercase letter "m" indicates that the adjacent nucleotide to the left of the "m" is a 2'-methoxy modified nucleotide; the lowercase letter "f" indicates that the adjacent nucleotide to the left is a 2'-fluoro modified nucleotide; the lowercase letter "s" between capital letters indicates that the linkage between the two adjacent nucleotides is a phosphorothioate linkage; "s" at the 3' end indicates that the end of the adjacent nucleotide to the left of the "s" is a phosphorothioate group. The synthesis of SA51 and the conjugate are described in Example 2.

**Table 2**

| Duplex No. | Sense strand sequence 5'-3' | SEQ ID NO: | Antisense strand sequence 5'-3' | SEQ ID NO: |
|---|---|---|---|---|
| SA012-00029 | UAUUUGCUGUCUUU AUAUU | 1 | AAUAUAAAGACAGCAA AUAUC | 13 |
| SA012-00031 | UUUGCUGUCUUUAU AUUCA | 2 | UGAAUAUAAAGACAGC AAAUA | 14 |
| SA012-00102 | AGAUGAGGAUAUUU GCUGU | 3 | ACAGCAAAUAUCCUCA UCUUU | 15 |
| SA012-00138 | AAGAUGAGGAUAUU UGCUA | 4 | UAGCAAAUAUCCUCAU CUUUC | 16 |
| SA012-00104 | AUGAGGAUAUUUGC UGUCU | 5 | AGACAGCAAAUAUCCU CAUCU | 17 |
| SA012-00150 | GAUAUUUGCUGUCU UUAUA | 6 | UAUAAAGACAGCAAAU AUCCU | 18 |
| SA012-00227 | CCAGAAAGAUGAGG AUAUU | 7 | AAUAUCCUCAUCUUUC UGGAA | 19 |
| SA012-00229 | GAGGAUAUUUGCUG UCUUU | 8 | AAAGACAGCAAAUAUC CUCAU | 20 |
| SA012-00272 | CUCAAUGCCUCAAU UUGUU | 9 | AACAAAUUGAGGCAUU GAGUG | 21 |
| SA012-00354 | UAUUUGCUGUCUUU AUAUA | 10 | UAUAUAAAGACAGCAA AUAUC | 22 |
| SA012-00355 | AGAUGAGGAUAUUU GCUGA | 11 | UCAGCAAAUAUCCUCA UCUUU | 23 |
| SA012-00356 | GAGGAUAUUUGCUG UCUUA | 12 | UAAGACAGCAAAUAUC CUCAU | 24 |
| SA012-00340 | GUUUUCUGCUUUCU GUCAA | 25 | UUGACAGAAAGCAGAA AACAA | 26 |
| SA012-00183 | GAGAUUAGAUCCUG AGGAA | 27 | UUCCUCAGGAUCUAAU CUCCU | 28 |

**Table 3**

| Duple x No. | Sense strand sequence 5'-3' | Antisense strand sequence 5'-3' |
|---|---|---|
| SD00 3141 | UmsAmsUmUmUmGmCfUfGfUmC mUmUmUmAmUmAmUmUm | AmsAfsUmAmUmAfAmAmGmAmCmAm GmCfAmAfAmUmAmsUmsCm |
| SD00 4135 | UmsAmsUmUmUmGmCfUfGfUmC mUmUmUmAmUmAmUmUm | AmsAfsUmAmUmAgoAmAmGmAmCmA mGmCfAmAfAmUmAmsUmsCm |
| SD00 4136 | UmsAmsUmUmUmGmCfUfGfUmC mUmUmUmAmUmAmUmUm | AmsAfsUmAmUmAfAgoAmGmAmCmA mGmCfAmAfAmUmAmsUmsCm |
| SD00 3143 | UmsUmsUmGmCmUmGfUfCfUmU mUmAmUmAmUmUmCmAm | UmsGfsAmAmUmAfUmAmAmAmGmAm CmAfGmCfAmAmAmsUmsAm |
| SD00 4137 | UmsUmsUmGmCmUmGfUfCfUmU mUmAmUmAmUmUmCmAm | UmsGfsAmAmUmAgoUmAmAmAmGmA mCmAfGmCfAmAmAmsUmsAm |
| SD00 4138 | UmsUmsUmGmCmUmGfUfCfUmU mUmAmUmAmUmUmCmAm | UmsGfsAmAmUmAfUgoAmAmAmGmA mCmAfGmCfAmAmAmsUmsAm |
| SD00 3187 | AmsGmsAmUmGmAmGfGfAfUmA mUmUmUmGmCmUmGmUm | AmsCfsAmGmCmAfAmAmUmAmUmCm CmUfCmAfUmCmUmsUmsUm |
| SD00 4139 | AmsGmsAmUmGmAmGfGfAfUmA mUmUmUmGmCmUmGmUm | AmsCfsAmGmCmAgoAmAmUmAmUmC mCmUfCmAfUmCmUmsUmsUm |
| SD00 4140 | AmsGmsAmUmGmAmGfGfAfUmA mUmUmUmGmCmUmGmUm | AmsCfsAmGmCmAfAgoAmUmAmUmCm CmUfCmAfUmCmUmsUmsUm |
| SD00 3223 | AmsAmsGmAmUmGmAfGfGfAmU mAmUmUmUmGmCmUmAm | UmsAfsGmCmAmAfAmUmAmUmCmCm UmCfAmUfCmUmUmsUmsCm |
| SD00 4143 | AmsAmsGmAmUmGmAfGfGfAmU mAmUmUmUmGmCmUmAm | UmsAfsGmCmAmAgoAmUmAmUmCmC mUmCfAmUfCmUmUmsUmsCm |
| SD00 4144 | AmsAmsGmAmUmGmAfGfGfAmU mAmUmUmUmGmCmUmAm | UmsAfsGmCmAmAfAgoUmAmUmCmCm UmCfAmUfCmUmUmsUmsCm |
| SD00 3189 | AmsUmsGmAmGmGmAfUfAfUmU mUmGmCmUmGmUmCmUm | AmsGfsAmCmAmGfCmAmAmAmUmAm UmCfCmUfCmAmUmsCmsUm |
| SD00 4142 | AmsUmsGmAmGmGmAfUfAfUmU mUmGmCmUmGmUmCmUm | AmsGfsAmCmAmGfCgoAmAmAmUmAm UmCfCmUfCmAmUmsCmsUm |
| SD00 3765 | GmsAmsUmAmUmUmUfGfCfUmG mUmCmUmUmUmAmUmAm | UmsAfsUmAmAmAfGmAmCmAmGmCm AmAfAmUfAmUmCmsCmsUm |
| SD00 4145 | GmsAmsUmAmUmUmUfGfCfUmG mUmCmUmUmUmAmUmAm | UmsAfsUmAmAmAgoGmAmCmAmGmC mAmAfAmUfAmUmCmsCmsUm |
| SD00 3842 | CmsCmsAmGmAmAmAfGfAfUmG mAmGmGmAmUmAmUmUm | AmsAfsUmAmUmCfCmUmCmAmUmCm UmUfUmCfUmGmGmsAmsAm |
| SD00 4129 | CmsCmsAmGmAmAmAfGfAfUmG mAmGmGmAmUmAmUmUm | AmsAfsUmAmUmCgoCmUmCmAmUmC mUmUfUmCfUmGmGmsAmsAm |
| SD00 4130 | CmsCmsAmGmAmAmAfGfAfUmG mAmGmGmAmUmAmUmUm | AmsAfsUmAmUmCfCgoUmCmAmUmCm UmUfUmCfUmGmGmsAmsAm |
| SD00 3844 | GmsAmsGmGmAmUmAfUfUfUmG mCmUmGmUmCmUmUmUm | AmsAfsAmGmAmCfAmGmCmAmAmAm UmAfUmCfCmUmCmsAmsUm |
| SD00 4131 | GmsAmsGmGmAmUmAfUfUfUmG mCmUmGmUmCmUmUmUm | AmsAfsAmGmAmCgoAmGmCmAmAmA mUmAfUmCfCmUmCmsAmsUm |
| SD00 4132 | GmsAmsGmGmAmUmAfUfUfUmG mCmUmGmUmCmUmUmUm | AmsAfsAmGmAmCfAgoGmCmAmAmAm UmAfUmCfCmUmCmsAmsUm |
| SD00 3887 | CmsUmsCmAmAmUmGfCfCfUmCm AmAmUmUmUmGmUmUm | AmsAfsCmAmAmAfUmUmGmAmGmGm CmAfUmUfGmAmGmsUmsGm |
| SD00 4133 | CmsUmsCmAmAmUmGfCfCfUmCm AmAmUmUmUmGmUmUm | AmsAfsCmAmAmAgoUmUmGmAmGmG mCmAfUmUfGmAmGmsUmsGm |
| SD00 4134 | CmsUmsCmAmAmUmGfCfCfUmCm AmAmUmUmUmGmUmUm | AmsAfsCmAmAmAfUgoUmGmAmGmG mCmAfUmUfGmAmGmsUmsGm |
| SD00 4740 | UmsAmsUmUmUmGmCfUfGfUmC mUmUmUmAmUmAmUmAm | UmsAfsUmAmUmAfAmAmGmAmCmAm GmCfAmAfAmUmAmsUmsCm |
| SD00 4741 | UmsAmsUmUmUmGmCfUfGfUmC mUmUmUmAmUmAmUmAm | UmsAfsUmAmUmAgoAmAmGmAmCmA mGmCfAmAfAmUmAmsUmsCm |
| SD00 4742 | UmsAmsUmUmUmGmCfUfGfUmC mUmUmUmAmUmAmUmAm | UmsAfsUmAmUmAfAgoAmGmAmCmA mGmCfAmAfAmUmAmsUmsCm |
| SD00 4743 | AmsGmsAmUmGmAmGfGfAfUmA mUmUmUmGmCmUmGmAm | UmsCfsAmGmCmAfAmAmUmAmUmCm CmUfCmAfUmCmUmsUmsUm |
| SD00 4744 | AmsGmsAmUmGmAmGfGfAfUmA mUmUmUmGmCmUmGmAm | UmsCfsAmGmCmAgoAmAmUmAmUmC mCmUfCmAfUmCmUmsUmsUm |
| SD00 4745 | AmsGmsAmUmGmAmGfGfAfUmA mUmUmUmGmCmUmGmAm | UmsCfsAmGmCmAfAgoAmUmAmUmCm CmUfCmAfUmCmUmsUmsUm |
| SD00 4746 | GmsAmsGmGmAmUmAfUfUfUmG mCmUmGmUmCmUmUmAm | UmsAfsAmGmAmCfAmGmCmAmAmAm UmAfUmCfCmUmCmsAmsUm |
| SD00 3955 | GmsUmsUmUmUmCmUfGfCfUmU mUmCmUmGmUmCmAmAm | UmsUfsGmAmCmAfGmAmAmAmGmCm AmGfAmAfAmAmCmsAmsAm |
| SD00 3798 | GmsAmsGmAmUmUmAfGfAfUmC mCmUmGmAmGmGmAmAm | UmsUfsCmCmUmCfAmGmGmAmUmCm UmAfAmUfCmUmCmsCmsUm |

**Table 4**

| Conjugate d Duplex No. | Sense strand sequence 5'-3' | Antisense strand sequence 5'-3' |
|---|---|---|
| SD004177 | UmsAmsUmUmUmGmCfUfGfUmCmU mUmUmAmUmAmUmAms-SA51- SA51-SA51 | VPUmsAfsUmAmUmAfAmAmGm AmCmAmGmCfAmAfAmUmAmsU msCm |
| SD004238 | UmsAmsUmUmUmGmCfUfGfUmCmU mUmUmAmUmAmUmAms-SA51- SA51-SA51 | VPUmsAfsUmAmUmAgoAmAmG mAmCmAmGmCfAmAfAmUmAms UmsCm |
| SD004239 | UmsAmsUmUmUmGmCfUfGfUmCmU mUmUmAmUmAmUmAms-SA51- SA51-SA51 | VPUmsAfsUmAmUmAfAgoAmGm AmCmAmGmCfAmAfAmUmAmsU msCm |
| SD004178 | UmsUmsUmGmCmUmGfUfCfUmUmU mAmUmAmUmUmCmAms-SA51- SA51-SA51 | VPUmsGfsAmAmUmAfUmAmAm AmGmAmCmAfGmCfAmAmAmsU msAm |
| SD004179 | UmsUmsUmGmCmUmGfUfCfUmUmU mAmUmAmUmUmCmAms-SA51- SA51-SA51 | VPUmsGfsAmAmUmAgoUmAmA mAmGmAmCmAfGmCfAmAmAms UmsAm |
| SD004240 | UmsUmsUmGmCmUmGfUfCfUmUmU mAmUmAmUmUmCmAms-SA51- SA51-SA51 | VPUmsGfsAmAmUmAfUgoAmAm AmGmAmCmAfGmCfAmAmAmsU msAm |
| SD004180 | AmsGmsAmUmGmAmGfGfAfUmAmU mUmUmGmCmUmGmAms-SA51- SA51-SA51 | VPUmsCfsAmGmCmAfAmAmUmA mUmCmCmUfCmAfUmCmUmsUm sUm |
| SD004241 | AmsGmsAmUmGmAmGfGfAfUmAmU mUmUmGmCmUmGmAms-SA51- SA51-SA51 | VPUmsCfsAmGmCmAgoAmAmUm AmUmCmCmUfCmAfUmCmUmsU msUm |
| SD004242 | AmsGmsAmUmGmAmGfGfAfUmAmU mUmUmGmCmUmGmAms-SA51- SA51-SA51 | VPUmsCfsAmGmCmAfAgoAmUm AmUmCmCmUfCmAfUmCmUmsU msUm |
| SD004181 | GmsAmsUmAmUmUmUfGfCfUmGmU mCmUmUmUmAmUmAms-SA51- SA51-SA51 | VPUmsAfsUmAmAmAfGmAmCm AmGmCmAmAfAmUfAmUmCmsC msUm |
| SD004243 | GmsAmsUmAmUmUmUfGfCfUmGmU mCmUmUmUmAmUmAms-SA51- SA51-SA51 | VPUmsAfsUmAmAmAgoGmAmCm AmGmCmAmAfAmUfAmUmCmsC msUm |
| SD004182 | GmsAmsGmGmAmUmAfUfUfUmGmC mUmGmUmCmUmUmAms-SA51- SA51-SA51 | VPUmsAfsAmGmAmCfAmGmCmA mAmAmUmAfUmCfCmUmCmsAm sUm |

### Example 4 In vitro screening for IC50: Dual-Glo^{®} Luciferase Assay

The activity of PD-L1-targeting siRNAs was tested in vitro using the Dual-Glo^{®} Luciferase Assay. The final concentration for the initial siRNA transfection was 10 nM. Compounds with an activity remaining percentage of less than 35% at the 10 nM concentration were subjected to activity screening at two concentrations, 1 nM and 0.2 nM. Preferred compounds selected from the two-concentration screening were subjected to IC50 screening.

### 1) Cell culture and plasmid/siRNA co-transfection

HEK293 cells were cultured in DMEM high glucose medium (Gibco, C11995500BT) with 10% fetal bovine serum at 37°C and 5% CO₂. When they reached about 90% confluence, they were digested with trypsin (Thermo, 25200072), and the cells were resuspended. The full-length human PD-L1 reference sequence (NM_014143.4) was cloned into the multiple cloning sites XhoI and NotI of the dual-luciferase psiCHECK2TM vector to construct the hs-psiCHECK plasmid. LipofectamineTM 2000 Transfection Reagent (Thermo, 11668500) was used to co-transfect the dual-luciferase plasmid and siRNA into 1x10⁴ cells. In a 96-well plate, 0.3 µL of LipofectamineTM 2000 was added to 19.7 µL of Opti-MEM (Gibco, 31985070) containing 20 ng of plasmid vector and siRNA for each well, and incubated at room temperature for 15 minutes. The mixture was added to the 96-well plate. Then, cells resuspended in 80 µL of fresh DMEM high glucose medium (Gibco, C11995500BT) with 10% fetal bovine serum were added. The cells were incubated for 24 hours, and luciferase activity was measured (Yeasen, 11405ES80).

### 2) Dual-Glo^{®} Luciferase Assay

24 hours after siRNA transfection, the dual luciferases, namely firefly luciferase (internal control) and Renilla luciferase (with the PD-L1 target sequence inserted after PolyA, fused into one mRNA), were measured. Following the product instruction manual (Yeasen, 11405ES80), the firefly luciferase luminescence signal was first detected on a multi-function microplate reader (Biotek, Synergy LX), and then the Renilla luciferase luminescence signal was detected. The detection sequence for Renilla luciferase on the microplate should be the same as that for firefly luciferase.

The Renilla luciferase signal reading for each well was normalized by the ratio to the firefly luciferase (control) signal, and then compared with cells transfected with the same plasmid but not treated with siRNA to evaluate the activity of each siRNA. All transfections were set up in duplicate.

The results are expressed as the percentage remaining relative to the control group without siRNA (control group is 100%), and the data are shown in Table 5. As seen from Table 5, except for SD003798 and SD003955, the experimental results of the other groups were better. siRNAs with better effects were further studied.

**Table 5**

| Compound No. | Hs-psiCHECK IC50 (nM) |
|---|---|
| SD003141 | 0.0168 |
| SD003143 | 0.0338 |
| SD003187 | 0.0174 |
| SD003189 | 0.0524 |
| SD003223 | 0.0263 |
| SD003765 | 0.0164 |
| SD003798 | 0.2531 |
| SD003842 | 0.0342 |
| SD003844 | 0.0286 |
| SD003887 | 0.0151 |
| SD003955 | 0.2154 |

### Example 5 Activity of siRNA in SNU-387 cells, in vitro IC50 screening

In vitro IC50 screening was carried out in SNU-387 cells using 8 concentrations (starting from 10 nM with 3-fold dilutions, for a total of 8 concentration points).

SNU-387 cells were cultured in RPMI-1640 medium (Thermo, 11875119) with 10% fetal bovine serum at 37°C and 5% CO₂. Then, they were digested with trypsin, and the cells were resuspended. RNAiMAX (Thermo, 13778150) was used to transfect siRNA into 2x10⁴ cells. In a 96-well plate, 0.3 µL of RNAiMAX was added to 19.7 µL of Opti-MEM (Gibco, 31985070) containing different concentrations of siRNA for each well, and incubated at room temperature for 15 minutes. The mixture was added to the 96-well plate. Then, cells resuspended in 80 µL of fresh RPMI-1640 medium (Thermo, 11875119) with 10% fetal bovine serum were added. The cells were incubated for 24 hours. RNA was extracted using a tissue and cell RNA extraction kit (Zhiang Bio, MNTR/FX96), reverse transcribed into cDNA (Takara, 6210B), and the expression level of the PD-L1 gene was measured by SYBR Green qPCR (Vazyme, Q711). The specific operating methods are described in the corresponding manuals.

Primers and probe for the target gene PD-L1:
Forward primer: TGCAGGGCATTCCAGAAAGAT (SEQ ID NO: 29);
Reverse primer: CCTTGGGAACCGTGACAGTA (SEQ ID NO: 30);

Primers for the internal reference gene β-Actin:
Forward primer: TGCACCACCAACTGCTTAGC (SEQ ID NO: 31);
Reverse primer: ACTGTGGTCATGAGTCCTTCCA (SEQ ID NO: 32);

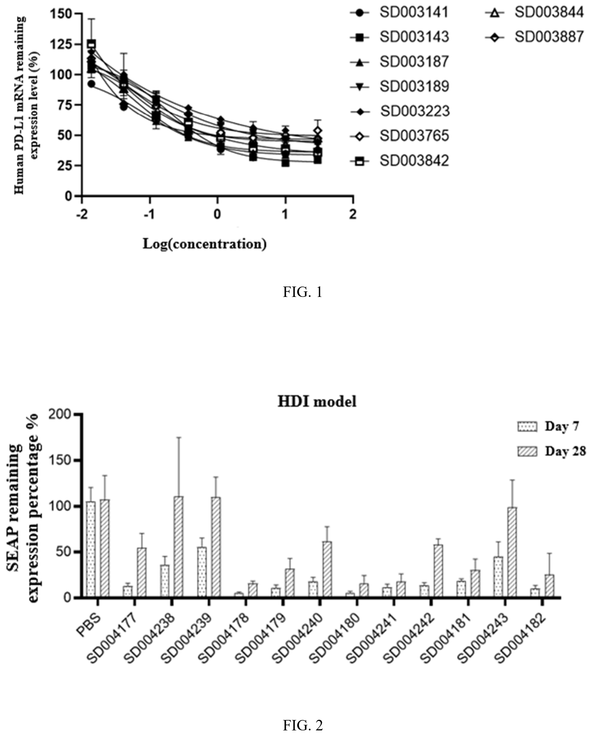

The relative level at different concentrations was calculated by comparison with cells not treated with siRNA, and the IC50 value was calculated. The results are shown in Table 6 and FIG. 1. The results showed that SD003141, SD003143, SD003187, SD003842, SD003844, and SD003887 had relatively good activity.

**Table 6**

| Compound No. | IC50 (nM) | Emax(%) |
|---|---|---|
| SD003141 | 0.32 | 68.17 |
| SD003143 | 0.53 | 72.72 |
| SD003187 | 0.34 | 65.89 |
| SD003189 | 3.66 | 57.75 |
| SD003223 | 12.76 | 55.34 |
| SD003765 | 19.35 | 52.04 |
| SD003842 | 0.78 | 63.95 |
| SD003844 | 0.94 | 57.36 |
| SD003887 | 0.83 | 52.52 |

### Example 6 In vitro on-target and off-target evaluation of siRNAs containing different chemical modifications

A dual-luciferase reporter gene assay was used to detect the on-target and off-target activity of siRNAs with different chemical modifications.

HEK293 cells were cultured in DMEM high glucose medium containing 10% fetal bovine serum at 37°C and 5% CO₂. Following the product instruction manual, Lipofectamine 2000 (ThermoFisher, 11668019) was used to co-transfect the reporter gene plasmid and siRNAs at different concentrations (starting from a final concentration of 90 nM, with 3-fold dilutions, for a total of 11 concentrations) into the cells. After 24 hours of transfection, detection was performed using a dual-luciferase detection kit (Yeasen, 11405ES80). A sequence complementary to the antisense strand (as the on-target reporter plasmid) and five tandem sequences consistent with positions 12-19 of the 5' end of the sense strand (as the off-target reporter plasmid) were inserted into the 3' untranslated region of the Renilla luciferase in the reporter plasmid, respectively. The reporter plasmid also contained firefly luciferase as an internal reference. The group transfected with only the plasmid served as the control group. The Renilla luciferase signal reading for each well was normalized by the ratio to the firefly luciferase (control) signal, and then compared with cells transfected with the same plasmid but not treated with siRNA to calculate the relative level at different concentrations and the IC50 value. The results are shown in Table 7. The results show that for some sequences, modifying the nucleotide at position 6 or 7 of the 5' region of the antisense strand does not affect activity while improving off-target effects, thus increasing the safety of the siRNA.

**Table 7**

| Compound No. | On-target | | Off-target | |
|---|---|---|---|---|
| | IC50(nM) | Emax | IC50(nM) | Emax |
| SD003141 | 0.016 | 86.05% | >90 | 27.60% |
| SD004135 | 0.034 | 86.25% | >90 | 13.80% |
| SD004136 | 0.022 | 80.20% | >90 | 18.40% |
| SD003143 | 0.021 | 81.35% | 0.429 | 83.85% |
| SD004137 | 0.025 | 85.50% | 0.996 | 73.25% |
| SD004138 | 0.051 | 80.00% | >90 | 44.60% |
| SD003187 | 0.013 | 85.95% | >90 | 8.50% |
| SD004139 | 0.032 | 85.50% | >90 | 12.15% |
| SD004140 | 0.034 | 84.50% | >90 | 16.85% |
| SD003223 | 0.114 | 79.10% | 4.577 | 67.55% |
| SD004143 | 0.098 | 82.80% | >90 | 45.25% |
| SD004144 | 0.312 | 80.75% | >90 | 10.05% |
| SD003189 | 0.092 | 78.90% | 0.358 | 89.90% |
| SD004142 | 0.054 | 73.60% | 6.663 | 55.70% |
| SD003765 | 0.029 | 74.85% | 3.795 | 57.30% |
| SD004145 | 0.055 | 79.10% | >90 | 43.25% |
| SD003842 | 0.019 | 78.25% | 0.037 | 88.10% |
| SD004129 | 0.032 | 81.95% | >90 | 53.90% |
| SD004130 | 0.029 | 80.75% | >90 | 51.80% |
| SD003844 | 0.064 | 75.75% | >90 | 52.85% |
| SD004131 | 0.063 | 77.70% | >90 | 48.75% |
| SD004132 | 0.127 | 68.55% | >90 | 45.60% |
| SD003887 | 0.071 | 70.65% | 1.865 | 61.45% |
| SD004133 | >10 | 50.50% | >90 | 22.90% |
| SD004134 | >10 | 46.80% | >90 | 25.75% |
| SD004740 | 0.006 | 90.10% | >90 | 27.95% |
| SD004741 | 0.015 | 88.95% | >90 | 22.85% |
| SD004742 | 0.013 | 86.95% | >90 | 29.10% |
| SD004743 | 0.016 | 89.55% | >90 | 20.30% |
| SD004744 | 0.011 | 93.50% | >90 | 15.00% |
| SD004745 | 0.024 | 90.35% | >90 | 19.75% |
| SD004746 | 0.001 | 88.70% | >90 | 25.50% |

### Example 7 In vivo activity of siRNAs containing different chemical modifications in HDI mice

In this example, based on the comprehensive results of in vitro on-target and off-target evaluations, siRNAs were selected for conjugate synthesis, and their in vivo activity was evaluated in an HDI mouse model. The siRNA conjugates were obtained by solid-phase synthesis as described in Example 2.

At least 14 days before conjugate administration, six to eight-week-old female Balb/C mice (Zhejiang Vital River Laboratory Animal Technology) were subjected to high-pressure tail vein injection to construct mice with humanized PD-L1 expression. 2 µg of a plasmid containing the PD-L1 mRNA sequence was injected into the mice via the tail vein within 5-7 seconds to generate PD-L1-SEAP model mice (Zhang G et al., "High levels of foreign gene expression in hepatocytes after tail vein injection of naked plasmid DNA." Human Gene Therapy 1999 Vol. 10, p1735-1737.). Inhibition of PD-L1 expression by the PD-L1 siRNA conjugate leads to the inhibition of secreted alkaline phosphatase (SEAP) expression. One day before administration, blood was collected via the orbital sinus, and serum was collected. The serum SEAP expression level was measured using the Phospha-Light SEAP Reporter Gene Assay System (Invitrogen) according to the product manual, and the mice were grouped according to the average SEAP level. Mice in the experimental group were given the conjugate, and mice in the vehicle group were given phosphate-buffered saline (PBS). Subcutaneous administration was performed at a dose of 3 mg/kg of the conjugate per mouse. Blood was collected again 7 days and 28 days after administration, and serum was collected. The serum SEAP expression level was measured using the Phospha-Light SEAP Reporter Gene Assay System (Invitrogen) according to the product manual. The SEAP level of each animal after administration was divided by the SEAP level of that animal before administration to determine the expression ratio "normalized to pre-treatment".

The results are expressed as the remaining expression level of serum SEAP in each group before and after administration (vehicle group is 100%). The relative remaining expression level results are shown in Table 8 and FIG. 2. At 7 days post-administration, conjugates SD004177, SD004178, SD004179, SD004240, SD004180, SD004241, SD004242, SD004181, and SD004182 showed better activity, with an inhibition rate greater than 80%. At 28 days post-administration, compounds SD004178 and SD004180 still maintained an activity greater than 80%, demonstrating long-lasting effect.

**Table 8**

| Compound | Remaining SEAP expression level on day 7 (%) | | Remaining SEAP expression level on day 28 (%) | |
|---|---|---|---|---|
| | AVG | SD | AVG | SD |
| PBS | 105.53 | 15.05 | 107.52 | 26.10 |
| SD004177 | 13.23 | 2.78 | 54.51 | 15.79 |
| SD004238 | 36.22 | 8.97 | 110.70 | 64.21 |
| SD004239 | 55.65 | 9.61 | 110.45 | 21.50 |
| SD004178 | 5.77 | 0.73 | 15.81 | 2.55 |
| SD004179 | 11.04 | 3.24 | 31.85 | 11.20 |
| SD004240 | 17.91 | 4.65 | 61.86 | 15.78 |
| SD004180 | 5.71 | 1.58 | 16.05 | 8.32 |
| SD004241 | 11.88 | 3.12 | 18.08 | 8.19 |
| SD004242 | 13.51 | 3.02 | 58.12 | 6.28 |
| SD004181 | 18.42 | 2.41 | 30.49 | 11.83 |
| SD004243 | 45.14 | 16.00 | 98.98 | 29.76 |
| SD004182 | 10.12 | 3.69 | 25.23 | 23.31 |

This example utilizes the HDI model to detect the level of human PD-L1 gene in vivo, which can better predict the in vivo activity of human PD-L1 mRNA, providing more and more reliable experimental data for clinical trials and drug marketing.

### Example 8 In vivo efficacy of siRNA in an AAV-HBV (hepatitis B virus) model in hPD-1/PD-L1 humanized mice

In this example, the in vivo efficacy of siRNA was evaluated in an AAV-HBV model in hPD-1/PD-L1 humanized mice to test its anti-hepatitis B virus activity. The siRNA conjugates were obtained by solid-phase synthesis as described in Example 2.

Six weeks before conjugate administration, an AAV-HBV model in hPD-1/PD-L1 humanized mice was established. A persistent HBV infection mouse model was prepared by intravenous tail injection of rAAV8-1.3HBV into 6-8 week-old B6-hPD-1/PD-L1 female mice. The AAV virus injection dose was 1E+11 vg/mouse. Six weeks after virus injection, blood was collected from the submandibular region of the animals. After blood collection, it was centrifuged at 5000 rpm, 4°C for 10 minutes. The serum was diluted with PBS, and the dilution factor needed to be determined according to the actual situation. Then, the diluted samples were sent to Beijing Dian Medical Laboratory Co., Ltd. for HBsAg (hepatitis B surface antigen) detection, and the measurement results were back-calculated according to the dilution factor. The HBsAg quantification method was direct electrochemiluminescence. The animals were grouped according to the HBsAg level. On day 0, the animals were injected subcutaneously, with an injection dose of 7.5 mg/kg body weight, once a week. At day 49 of the experiment, blood was collected to detect the HBsAg level to evaluate the anti-hepatitis B virus activity of the siRNA.

The reduction in HBsAg at 49 days post-administration is shown in Table 9. The results indicate that both SD004178 and SD004180 can significantly reduce the HBsAg level.

**Table 9**

| Compound No. | HBsAg reduction at 49 days post-administration log10 | |
|---|---|---|
| | AVG | SD |
| PBS | -0.25 | 0.49 |
| SD004180 | -1.66 | 0.07 |
| SD004178 | -1.62 | 0.08 |

The present invention has been described in detail above. The purpose is to enable those skilled in the art to understand the content of the present invention and implement it. This cannot be used to limit the protection scope of the present invention. Any equivalent changes or modifications made according to the spirit and substance of the present invention should be covered within the protection scope of the present invention.

## Claims

1. An siRNA, comprising a sense strand and an antisense strand, wherein: each nucleotide in the siRNA is independently a modified or unmodified nucleotide, the sense strand comprises a nucleotide sequence selected from one of the nucleotide sequences as set forth in SEQ ID NOs: 1 to 12 or a nucleotide sequence with no more than 3 base mutations thereto, and the antisense strand comprises a nucleotide sequence selected from one of the nucleotide sequences as set forth in SEQ ID NOs: 13 to 24 or a nucleotide sequence with no more than 5 base mutations thereto.

2. The siRNA of claim 1, wherein in a 5' to 3' direction, the base mutation in the sense strand is at the 3' end of the nucleotide sequence thereof, and the base mutation in the antisense strand is at one or more positions within the nucleotide sequence, selected from the 5' end, positions 2 to 8, and the last two nucleotides at the 3' end.

3. The siRNA of claim 1, wherein the modified nucleotide is one or a combination of more than one selected from the group consisting of a 2'-methoxy modified nucleotide, a 2'-fluoro modified nucleotide, a 2'-O-CH₂-CH₂-O-CH₃ modified nucleotide, a 2'-O-CH₂-CH=CH₂ modified nucleotide, a 2'-CH₂-CH₂-CH=CH₂ modified nucleotide, a 2'-deoxynucleotide, a 2'-methoxyethyl modified nucleotide, a phosphorothioate-modified nucleotide, a VP-modified nucleotide, LNA, ENA, cET BNA, UNA, GNA, and 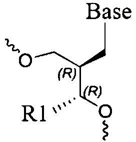 , wherein R1 is H, OH, or CH₃, and Base is a natural nucleobase, a modified nucleobase, a universal base, or an H atom.

4. The siRNA of any one of claims 1 to 3, wherein one or more nucleotides in the sense strand or the antisense strand are modified nucleotides.

5. The siRNA of any one of claims 1 to 3, wherein in a 5' to 3' direction, the nucleotides at positions 7, 8, and 9 of the sense strand are 2'-fluoro modified nucleotides, and the nucleotides at other positions of the sense strand are 2'-methoxy modified nucleotides; in a 5' to 3' direction, the nucleotides at positions 2, 14, and 16 of the antisense strand are 2'-fluoro modified nucleotides, and the nucleotides at positions of the antisense strand other than positions 2, 6, 7, 14, and 16 are 2'-methoxy modified nucleotides; at least one linkage between the following nucleotides in the siRNA is a phosphorothioate linkage:
the linkage between the 1st nucleotide and the 2nd nucleotide from the 5' end of the sense strand;
the linkage between the 2nd nucleotide and the 3rd nucleotide from the 5' end of the sense strand;
the linkage between the 1st nucleotide and the 2nd nucleotide from the 3' end of the sense strand;
the linkage between the 2nd nucleotide and the 3rd nucleotide from the 3' end of the sense strand;
the linkage between the 1st nucleotide and the 2nd nucleotide from the 5' end of the antisense strand;
the linkage between the 2nd nucleotide and the 3rd nucleotide from the 5' end of the antisense strand;
the linkage between the 1st nucleotide and the 2nd nucleotide from the 3' end of the antisense strand;
the linkage between the 2nd nucleotide and the 3rd nucleotide from the 3' end of the antisense strand;
the 3' end of the sense strand is a phosphorothioate group, and the 5' end of the antisense strand is a VP-modified nucleotide.

6. The siRNA of any one of claims 1 to 3, wherein in a 5' to 3' direction, the nucleotide at position 6 of the antisense strand is a 2'-fluoro modified nucleotide or 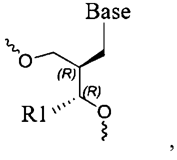 and the nucleotide at position 7 of the antisense strand is a 2'-methoxy modified nucleotide or 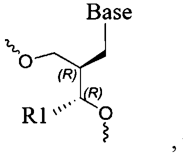 wherein R1 is H, OH, or CH₃, and Base is a natural nucleobase, a modified nucleobase, a universal base, or an H atom.

7. The siRNA of claim 6, wherein the nucleotide at position 6 or position 7 of the antisense strand is 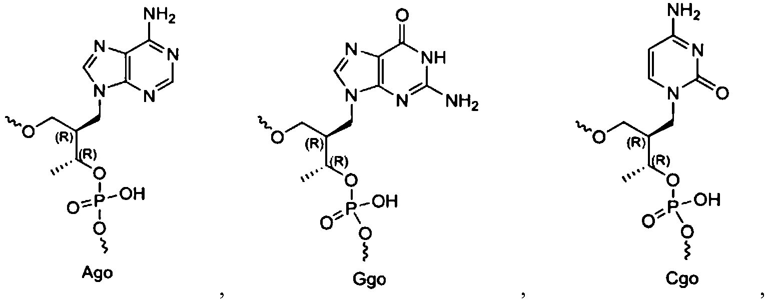 or

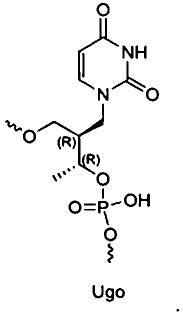

8. The siRNA of claim 1, wherein the siRNA is any one of the sequences as shown in the table below:
| Duplex No. | Sense strand sequence 5'-3' | Antisense strand sequence 5'-3' |
|---|---|---|
| SA012-00029 | UAUUUGCUGUCUUUAUAUU | AAUAUAAAGACAGCAAAUAUC |
| SA012-00031 | UUUGCUGUCUUUAUAUUCA | UGAAUAUAAAGACAGCAAAUA |
| SD003141 | UmsAmsUmUmUmGmCfUfGfUm CmUmUmUmAmUmAmUmUm | AmsAfsUmAmUmAfAmAmGmAmCm AmGmCfAmAfAmUmAmsUmsCm |
| SD003143 | UmsUmsUmGmCmUmGfUfCfUm UmUmAmUmAmUmUmCmAm | UmsGfsAmAmUmAfUmAmAmAmGm AmCmAfGmCfAmAmAmsUmsAm |

9. The siRNA of claim 1, wherein the siRNA is any one of the sequences as shown in the table below:
| Duplex No. | Sense strand sequence 5'-3' | Antisense strand sequence 5'-3 |
|---|---|---|
| SA012-00102 | AGAUGAGGAUAUUUGCUGU | ACAGCAAAUAUCCUCAUCUUU |
| SA012-00138 | AAGAUGAGGAUAUUUGCUA | UAGCAAAUAUCCUCAUCUUUC |
| SA012-00104 | AUGAGGAUAUUUGCUGUCU | AGACAGCAAAUAUCCUCAUCU |
| SA012-00150 | GAUAUUUGCUGUCUUUAUA | UAUAAAGACAGCAAAUAUCCU |
| SA012-00227 | CCAGAAAGAUGAGGAUAUU | AAUAUCCUCAUCUUUCUGGAA |
| SA012-00229 | GAGGAUAUUUGCUGUCUUU | AAAGACAGCAAAUAUCCUCAU |
| SA012-00272 | CUCAAUGCCUCAAUUUGUU | AACAAAUUGAGGCAUUGAGUG |
| SD003187 | AmsGmsAmUmGmAmGfGfAfUm AmUmUmUmGmCmUmGmUm | AmsCfsAmGmCmAfAmAmUmAmUmC mCmUfCmAfUmCmUmsUmsUm |
| SD003223 | AmsAmsGmAmUmGmAfGfGfAm UmAmUmUmUmGmCmUmAm | UmsAfsGmCmAmAfAmUmAmUmCmC mUmCfAmUfCmUmUmsUmsCm |
| SD003189 | AmsUmsGmAmGmGmAfUfAfUm UmUmGmCmUmGmUmCmUm | AmsGfsAmCmAmGfCmAmAmAmUm AmUmCfCmUfCmAmUmsCmsUm |
| SD003765 | GmsAmsUmAmUmUmUfGfCfUmG mUmCmUmUmUmAmUmAm | UmsAfsUmAmAmAfGmAmCmAmGm CmAmAfAmUfAmUmCmsCmsUm |
| SD003842 | CmsCmsAmGmAmAmAfGfAfUmG mAmGmGmAmUmAmUmUm | AmsAfsUmAmUmCfCmUmCmAmUmC mUmUfUmCfUmGmGmsAmsAm |
| SD003844 | GmsAmsGmGmAmUmAfUfUfUm GmCmUmGmUmCmUmUmUm | AmsAfsAmGmAmCfAmGmCmAmAm AmUmAfUmCfCmUmCmsAmsUm |
| SD003887 | CmsUmsCmAmAmUmGfCfCfUmC mAmAmUmUmUmGmUmUm | AmsAfsCmAmAmAfUmUmGmAmGm GmCmAfUmUfGmAmGmsUmsGm |

10. The siRNA of claim 1, wherein the siRNA is any one of the sequences as shown in the table below:
| Duplex No. | Sense strand sequence 5'-3' | Antisense strand sequence 5'-3' |
|---|---|---|
| SA012-00354 | UAUUUGCUGUCUUUAUAUA | UAUAUAAAGACAGCAAAUAUC |
| SA012-00355 | AGAUGAGGAUAUUUGCUGA | UCAGCAAAUAUCCUCAUCUUU |
| SA012-00356 | GAGGAUAUUUGCUGUCUUA | UAAGACAGCAAAUAUCCUCAU |
| SD004135 | UmsAmsUmUmUmGmCfUfGfUm CmUmUmUmAmUmAmUmUm | AmsAfsUmAmUmAgoAmAmGmAmC mAmGmCfAmAfAmUmAmsUmsCm |
| SD004136 | UmsAmsUmUmUmGmCfUfGfUm CmUmUmUmAmUmAmUmUm | AmsAfsUmAmUmAfAgoAmGmAmCm AmGmCfAmAfAmUmAmsUmsCm |
| SD004137 | UmsUmsUmGmCmUmGfUfCfUm UmUmAmUmAmUmUmCmAm | UmsGfsAmAmUmAgoUmAmAmAmG mAmCmAfGmCfAmAmAmsUmsAm |
| SD004138 | UmsUmsUmGmCmUmGfUfCfUm UmUmAmUmAmUmUmCmAm | UmsGfsAmAmUmAfUgoAmAmAmGm AmCmAfGmCfAmAmAmsUmsAm |
| SD004139 | AmsGmsAmUmGmAmGfGfAfUm AmUmUmUmGmCmUmGmUm | AmsCfsAmGmCmAgoAmAmUmAmUm CmCmUfCmAfUmCmUmsUmsUm |
| SD004140 | AmsGmsAmUmGmAmGfGfAfUm AmUmUmUmGmCmUmGmUm | AmsCfsAmGmCmAfAgoAmUmAmUm CmCmUfCmAfUmCmUmsUmsUm |
| SD004143 | AmsAmsGmAmUmGmAfGfGfAm UmAmUmUmUmGmCmUmAm | UmsAfsGmCmAmAgoAmUmAmUmCm CmUmCfAmUfCmUmUmsUmsCm |
| SD004144 | AmsAmsGmAmUmGmAfGfGfAm UmAmUmUmUmGmCmUmAm | UmsAfsGmCmAmAfAgoUmAmUmCm CmUmCfAmUfCmUmUmsUmsCm |
| SD004142 | AmsUmsGmAmGmGmAfUfAfUm UmUmGmCmUmGmUmCmUm | AmsGfsAmCmAmGfCgoAmAmAmUm AmUmCfCmUfCmAmUmsCmsUm |
| SD004145 | GmsAmsUmAmUmUmUfGfCfUm GmUmCmUmUmUmAmUmAm | UmsAfsUmAmAmAgoGmAmCmAmG mCmAmAfAmUfAmUmCmsCmsUm |
| SD004129 | CmsCmsAmGmAmAmAfGfAfUm GmAmGmGmAmUmAmUmUm | AmsAfsUmAmUmCgoCmUmCmAmUm CmUmUfUmCfUmGmGmsAmsAm |
| SD004130 | CmsCmsAmGmAmAmAfGfAfUm GmAmGmGmAmUmAmUmUm | AmsAfsUmAmUmCfCgoUmCmAmUm CmUmUfUmCfUmGmGmsAmsAm |
| SD004131 | GmsAmsGmGmAmUmAfUfUfUm GmCmUmGmUmCmUmUmUm | AmsAfsAmGmAmCgoAmGmCmAmAm AmUmAfUmCfCmUmCmsAmsUm |
| SD004132 | GmsAmsGmGmAmUmAfUfUfUm GmCmUmGmUmCmUmUmUm | AmsAfsAmGmAmCfAgoGmCmAmAm AmUmAfUmCfCmUmCmsAmsUm |
| SD004133 | CmsUmsCmAmAmUmGfCfCfUm CmAmAmUmUmUmGmUmUm | AmsAfsCmAmAmAgoUmUmGmAmG mGmCmAfUmUfGmAmGmsUmsGm |
| SD004134 | CmsUmsCmAmAmUmGfCfCfUm CmAmAmUmUmUmGmUmUm | AmsAfsCmAmAmAfUgoUmGmAmGm GmCmAfUmUfGmAmGmsUmsGm |
| SD004740 | UmsAmsUmUmUmGmCfUfGfUm CmUmUmUmAmUmAmUmAm | UmsAfsUmAmUmAfAmAmGmAmCm AmGmCfAmAfAmUmAmsUmsCm |
| SD004741 | UmsAmsUmUmUmGmCfUfGfUm CmUmUmUmAmUmAmUmAm | UmsAfsUmAmUmAgoAmAmGmAmC mAmGmCfAmAfAmUmAmsUmsCm |
| SD004742 | UmsAmsUmUmUmGmCfUfGfUm CmUmUmUmAmUmAmUmAm | UmsAfsUmAmUmAfAgoAmGmAmCm AmGmCfAmAfAmUmAmsUmsCm |
| SD004743 | AmsGmsAmUmGmAmGfGfAfUm AmUmUmUmGmCmUmGmAm | UmsCfsAmGmCmAfAmAmUmAmUmC mCmUfCmAfUmCmUmsUmsUm |
| SD004744 | AmsGmsAmUmGmAmGfGfAfUm AmUmUmUmGmCmUmGmAm | UmsCfsAmGmCmAgoAmAmUmAmUm CmCmUfCmAfUmCmUmsUmsUm |
| SD004745 | AmsGmsAmUmGmAmGfGfAfUm AmUmUmUmGmCmUmGmAm | UmsCfsAmGmCmAfAgoAmUmAmUm CmCmUfCmAfUmCmUmsUmsUm |
| SD004746 | GmsAmsGmGmAmUmAfUfUfUm GmCmUmGmUmCmUmUmAm | UmsAfsAmGmAmCfAmGmCmAmAm AmUmAfUmCfCmUmCmsAmsUm |

11. An siRNA conjugate, comprising one or more of the siRNAs of any one of claims 1 to 10, and a conjugating group conjugated to the siRNA.

12. The siRNA conjugate of claim 11, wherein the conjugating group is derived from compound SA51, the number of compound SA51 units in the conjugating group is 2 to 4, and the 2 to 4 compound SA51 units are sequentially linked by chemical bonds; and the structural formula of the compound SA51 is:

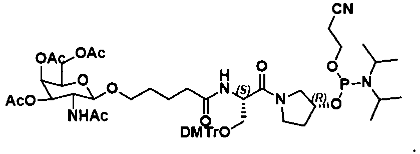

13. The siRNA conjugate of claim 11 or 12, wherein the conjugating group is linked to the 3' end and/or the 5' end of the sense strand.

14. The siRNA conjugate of claim 11, wherein the siRNA conjugate has a structural formula as follows: 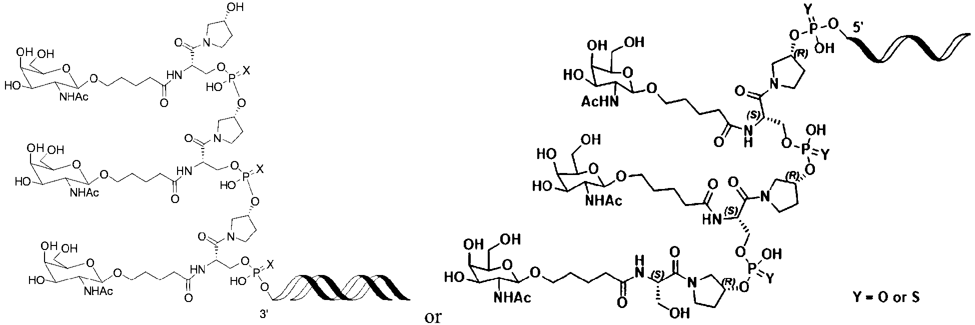 wherein, 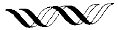 represents a duplex formed by the siRNA of the present disclosure, and X is O or S.

15. A pharmaceutical composition, comprising the siRNA of any one of claims 1 to 10 or the siRNA conjugate of any one of claims 11 to 14, and a pharmaceutically acceptable carrier or excipient.

16. The pharmaceutical composition of claim 15, wherein the pharmaceutical composition is for inhibiting programmed cell death ligand-1 gene expression.

17. Use of the siRNA of any one of claims 1 to 10, the siRNA conjugate of any one of claims 11 to 14, or the pharmaceutical composition of claim 15 or 16 for preparing a medicament for treating and/or preventing a disease associated with PD-L1 gene expression.

18. The use of claim 17, wherein the disease is viral hepatitis or cancer.

19. The use of claim 18, wherein the viral hepatitis is caused by infection with hepatitis B virus, hepatitis C virus, or hepatitis D virus.
